# EUROPEAN PATENT APPLICATION

(11) **EP 1 686 379 A1**
(43) Date of publication of application: **02.08.2006**
(21) Application number: 04792813.0
(22) Date of filing: 15.10.2004
(51) Int. Cl.: G01N 33/68, G01N 33/483, G01N 33/84, C12N 15/11, C12N 1/15, C12N 1/19, C12N 1/21, C12N 5/10, C07K 14/435, C07K 19/00, C12Q 1/02

(54) **FLUORESCENCE INDICATOR UTILIZING FRET**

(30) Priority: 15.10.2003 JP 2003355192
(71) Applicant: RIKEN, Wako-shi, Saitama 351-0198 (JP); Japan Science and Technology Agency, Kawaguchi-shi, Saitama 332-0012 (JP)
(72) Inventor: MIYAWAKI, Atsushi, Riken, Wako-shi, Saitama 351-0198 (JP); NAGAI, Takeharu, Itabashi-ku, Tokyo 174-0063 (JP)
(74) Representative: Sternagel, Fleischer, Godemeyer & Partner
(86) International application number: PCT/JP2004/015671
(87) International publication number: WO 2005/036178

(57) **Abstract**

It is an object of the present invention to provide a novel fluorescent indicator used for analyzing intermolecular interaction or a change in the intramolecular structure, using fluorescence resonance energy transfer (FRET). The present invention provides a fluorescent indicator, which has a structure such that a donor fluorescent protein and an acceptor fluorescent protein bind to both termini of the target sequence of an analytical substance, wherein the analytical substance binds to or acts on said target sequence, so that the three-dimensional structure of the indicator can be changed, thereby generating fluorescence resonance energy transfer (FRET), said fluorescent indicator being **characterized in that** the said donor fluorescent protein and/or said acceptor fluorescent protein are circularly permuted fluorescent proteins obtained by substituting the amino acid sequence on the N-terminal side of a wild-type fluorescent protein or a mutant protein thereof with the amino acid sequence on the C-terminal side thereof, and in that the thus obtained fluorescent proteins have a fluorescent peak wavelength substantially identical to that of a florescent protein, which has not yet been subjected to the circular permutation.

SEQUENCE LISTING
<110> RIKEN
<110> JAPAN SCIENCE AND TECHNOLOGY AGENCY
<120> A fluorescent indicator using FRET
<130> A41654A
<160> 46
<210> 1
<211> 26
<212> PRT
<213> animal
<400> 1
Lys Arg Arg Trp Lys Lys Asn Phe Ile Ala Val Ser Ala Ala Asn Arg
1               5                   10                  15
Phe Lys Lys Ile Ser Ser Ser Gly Ala Leu
            20                  25
<210> 2
<211> 20
<212> PRT
<213> animal
<400> 2
Ala Arg Arg Lys Trp Gln Lys Thr Gly His Ala Val Arg Ala Ile Gly
1               5                   10                  15
Arg Leu Ser Ser
            20
<210> 3
<211> 20
<212> PRT

<213> animal
<400> 3
Ala Arg Arg Lys Leu Lys Gly Ala Ile Leu Thr Thr Met Leu Ala Thr
1               5                   10                  15
Arg Asn Phe Ser
            20
<210> 4
<211> 17
<212> PRT
<213> animal
<400> 4
Gly Val Arg Asn Ile Lys Ser Met Trp Glu Lys Gly Asn Val Phe Ser
1               5                   10                  15
Ser
<210> 5
<211> 20
<212> PRT
<213> animal
<400> 5
Ala Arg Arg Lys Leu Lys Ala Ala Val Lys Ala Val Val Ala Ser Ser
1               5                   10                  15
Arg Leu Gly Ser
            20
<210> 6
<211> 26
<212> PRT
<213> animal

<400> 6

Phe Met Asn Asn Trp Glu Val Tyr Lys Leu Leu Ala His Ile Arg Pro

1               5                   10                  15

Pro Ala Pro Lys Ser Gly Ser Tyr Thr Val

            20                  25

<210> 7

<211> 24

<212> PRT

<213> animal

<400> 7

Ala Arg Lys Glu Val Ile Arg Asn Lys Ile Arg Ala Ile Gly Lys Met

1               5                   10                  15

Ala Arg Val Phe Ser Val Leu Arg

            20

<210> 8

<211> 26

<212> PRT

<213> animal

<400> 8

Leu Arg Arg Leu Ile Asp Ala Tyr Ala Phe Arg Ile Tyr Gly His Trp

1               5                   10                  15

Val Lys Lys Gly Gln Gln Gln Asn Arg Gly

            20                  25

<210> 9

<211> 27

<212> PRT

<213> animal

<400> 9

Arg Gly Lys Phe Lys Val Ile Cys Leu Thr Val Leu Ala Ser Val Arg

1               5                   10                  15

Ile Tyr Tyr Gln Tyr Arg Arg Val Lys Pro Gly

            20                  25

<210> 10

<211> 28

<212> PRT

<213> animal

<400> 10

Leu Arg Arg Gly Gln Ile Leu Trp Phe Arg Gly Leu Asn Arg Ile Gln

1               5                   10                  15

Thr Gln Ile Lys Val Val Asn Ala Phe Ser Ser Ser

            20                  25

<210> 11

<211> 21

<212> PRT

<213> animal

<400> 11

Arg Arg Lys His Leu Gln Arg Pro Ile Phe Arg Leu Arg Cys Leu Val

1               5                   10                  15

Lys Gln Leu Glu Lys

            20

<210> 12

<211> 21

<212> PRT

<213> animal

<400> 12

Thr Glu Lys Met Trp Gln Arg Leu Lys Gly Ile Leu Arg Cys Leu Val

1               5                   10                  15

Lys Gln Leu Glu Lys

            20

<210> 13

<211> 23

<212> PRT

<213> animal

<400> 13

Lys Arg Arg Ala Ile Gly Phe Lys Lys Leu Ala Glu Ala Val Lys Phe

1               5                   10                  15

Ser Ala Lys Leu Met Gly Gln

            20

<210> 14

<211> 28

<212> PRT

<213> animal

<400> 14

Ile Lys Pro Ala Lys Arg Met Lys Phe Lys Thr Val Cys Tyr Leu Leu

1               5                   10                  15

Val Gln Leu Met His Cys Arg Lys Met Phe Lys Ala

            20                  25

<210> 15

<211> 22

<212> PRT

<213> animal

<400> 15

Ala Cys Ile Asp Leu Leu Trp Lys Ile Ala Arg Ala Gly Ala Arg Ser

1               5                   10                  15

Ala Val Gly Thr Glu Ala

            20

<210> 16

<211> 27

<212> PRT

<213> animal

<400> 16

Lys Ala His Lys Ala Ala Thr Lys Ile Gln Ala Ser Phe Arg Gly His

1               5                   10                  15

Ile Thr Arg Lys Lys Leu Lys Gly Glu Lys Lys

            20                  25

<210> 17

<211> 24

<212> PRT

<213> animal

<400> 17

Lys Thr Ala Ser Pro Trp Lys Ser Ala Arg Leu Met Val His Thr Val

1               5                   10                  15

Ala Thr Phe Asn Ser Ile Lys Glu

            20

<210> 18

<211> 25

<212> PRT

<213> animal

<400> 18

Lys Lys Lys Lys Lys Arg Phe Ser Phe Lys Lys Ser Phe Lys Leu Ser

1               5                   10                  15

Gly Phe Ser Phe Lys Lys Ser Lys Lys

            20                  25

<210> 19

<211> 24

<212> PRT

<213> animal

<400> 19

Lys Lys Lys Lys Lys Phe Ser Phe Lys Lys Pro Phe Lys Leu Ser Gly

1               5                   10                  15

Leu Ser Phe Lys Arg Asn Arg Lys

            20

<210> 20

<211> 31

<212> PRT

<213> animal

<400> 20

Lys Gln Gln Lys Glu Lys Thr Arg Trp Leu Asn Thr Pro Asn Thr Tyr

1               5                   10                  15

Leu Arg Val Asn Val Ala Asp Glu Val Gln Arg Asn Met Gly Ser

            20                  25                  30

<210> 21

<211> 21

<212> PRT

<213> animal

<400> 21

<210> 22

<211> 21

<212> PRT

<213> animal

<400> 22

Tyr His Arg Leu Arg Asp Leu Leu Leu Ile Val Lys Arg Ile Val Glu

1               5                   10                  15

Leu Leu Gly Arg Arg

            20

<210> 23

<211> 23

<212> PRT

<213> animal

<400> 23

Gln Gln Leu Ala Thr Leu Ile Gln Lys Thr Tyr Arg Gly Trp Arg Cys

1               5                   10                  15

Arg Thr His Tyr Gln Leu Met

            20

<210> 24

<211> 24

<212> PRT

<213> animal

<400> 24

Arg Ala Ala Cys Ile Arg Ile Gln Lys Thr Ile Arg Gly Trp Leu Leu

1               5                   10                  15

Arg Lys Arg Tyr Leu Cys Met Gln

            20

<210> 25

<211> 12

<212> PRT

<213> animal

<400> 25

Ile Asn Leu Lys Ala Ala Leu Ala Lys Lys Ile Leu

1               5                   10

<210> 26

<211> 26

<212> PRT

<213> animal

<400> 26

Gly Ile Gly Ala Val Leu Lys Val Leu Thr Thr Gly Leu Pro Ala Leu

1               5                   10                  15

Ile Ser Trp Ile Lys Arg Lys Arg Gln Gln

            20                  25

<210> 27

<211> 30

<212> PRT

<213> animal

<400> 27

His Ser Gln Gly Thr Phe Thr Thr Ser Asp Tyr Ser Lys Tyr Leu Asp

1                   5                   10                  15

Ser Arg Arg Ala Gln Asp Phe Val Gln Trp Leu Met Asn Thr

                20                  25                  30

<210> 28

<211> 27

<212> PRT

<213> animal

<400> 28

His Ser Asp Gly Thr Phe Thr Ser Glu Leu Ser Arg Leu Arg Asp Ser

  1                   5                   10                  15

Ala Arg Leu Gln Arg Leu Leu Gln Gly Leu Val

                20                  25

<210> 29

<211> 28

<212> PRT

<213> animal

<400> 29

His Ser Asp Ala Val Phe Thr Asp Asn Tyr Thr Arg Leu Arg Lys Gln

  1                   5                   10                  15

Met Ala Val Lys Lys Tyr Leu Asn Ser Ile Leu Asn

                20                  25

<210> 30

<211> 33

<212> PRT

<213> animal

<400> 20

Tyr Ala Asp Gly Thr Phe Ile Ser Asp Tyr Ser Ala Ile Met Asn Lys
1               5                   10                  15
Ile Arg Gln Gln Asp Phe Val Asn Trp Leu Leu Ala Gln Gln Gln Lys
            20                  25                  30
Ser

<210> 31
<211> 17
<212> PRT
<213> animal
<400> 31
Lys Leu Trp Lys Lys Leu Leu Lys Leu Leu Lys Lys Leu Leu Lys Leu
1               5                   10                  15
Gly

<210> 32
<211> 5
<212> PRT
<213> eucaryotic cell
<400> 32
Lys Lys Lys Arg Lys
                5
<210> 33
<211> 26
<212> PRT
<213> eucaryotic cell
<400> 33

Met Leu Arg Thr Ser Ser Leu Phe Thr Arg Arg Val Gln Pro Ser Leu
1               5                   10                  15
Phe Arg Asn Ile Leu Arg Leu Gln Ser Thr
            20                  25
<210> 34
<211> 4
<212> PRT
<213> eucaryotic cell
<400> 34
Lys Asp Glu Leu

<210> 35
<211> 3
<212> PRT
<213> eucaryotic cell
<400> 35
Ser Lys Leu

<210> 36
<211> 4
<212> PRT
<213> eucaryotic cell
<220>
<222> (4)
<223> any amino acid
<400> 36
Cys Ala Ala Xaa

<210> 37

<211> 2

<212> PRT

<213> eucaryotic cell

<400> 37

Cys Cys

<210> 38

<211> 3

<212> PRT

<213> eucaryotic cell

<220>

<222> (2)

<223> any amino acid

<400> 38

Cys Xaa Cys

<210> 39

<211> 4

<212> PRT

<213> eucaryotic cell

<220>

<222> (3), (4)

<223> any amino acid

<400> 39

Cys Cys Xaa Xaa

<210> 40

<211> 16

<212> PRT

<213> Artificial Sequence

<400> 40

Gly Thr Gly Gly Ser Gly Gly Gly Thr Gly Gly Ser Gly Gly Gly Thr

1               5                   10                  15

<210> 41

<211> 647

<212> PRT

<213> Artificial Sequence

<400> 41

Met Val Ser Lys Gly Glu Glu Leu Phe Thr Gly Val Val Pro Ile Leu

1               5                   10                  15

Val Glu Leu Asp Gly Asp Val Asn Gly His Arg Phe Ser Val Ser Gly

            20                  25                  30

Glu Gly Glu Gly Asp Ala Thr Tyr Gly Lys Leu Thr Leu Lys Phe Ile

        35                  40                  45

Cys Thr Thr Gly Lys Leu Pro Val Pro Trp Pro Thr Leu Val Thr Thr

    50                  55                  60

Leu Thr Trp Gly Val Gln Cys Phe Ser Arg Tyr Pro Asp His Met Lys

65                  70                  75                  80

Gln His Asp Phe Phe Lys Ser Ala Met Pro Glu Gly Tyr Val Gln Glu

                85                  90                  95

Arg Thr Ile Phe Phe Lys Asp Asp Gly Asn Tyr Lys Thr Arg Ala Glu

            100                 105                 110
Val Lys Phe Glu Gly Asp Thr Leu Val Asn Arg Ile Glu Leu Lys Gly
        115                 120                 125
Ile Asp Phe Lys Glu Asp Gly Asn Ile Leu Gly His Lys Leu Glu Tyr
    130                 135                 140
Asn Tyr Ile Ser His Asn Val Tyr Ile Thr Ala Asp Lys Gln Lys Asn
145                 150                 155                 160
Gly Ile Lys Ala Asn Phe Lys Ile Arg His Asn Ile Glu Asp Gly Ser
                165                 170                 175
Val Gln Leu Ala Asp His Tyr Gln Gln Asn Thr Pro Ile Gly Asp Gly
            180                 185                 190
Pro Val Leu Leu Pro Asp Asn His Tyr Leu Ser Thr Gln Ser Ala Leu
        195                 200                 205
Ser Lys Asp Pro Lys Glu Lys Arg Asp His Met Val Leu Leu Glu Phe
    210                 215                 220
Val Thr Ala Ala Arg Met His Asp Gln Leu Thr Glu Glu Gln Ile Ala
225                 230                 235                 240
Glu Phe Lys Glu Ala Phe Ser Leu Phe Asp Lys Asp Gly Asp Gly Thr
                245                 250                 255
Ile Thr Thr Lys Glu Leu Gly Thr Val Met Arg Ser Leu Gly Gln Asn
            260                 265                 270
Pro Thr Glu Ala Glu Leu Gln Asp Met Ile Asn Glu Val Asp Ala Asp
        275                 280                 285
Gly Asn Gly Thr Ile Tyr Phe Pro Glu Phe Leu Thr Met Met Ala Arg
    290                 295                 300
Lys Met Lys Asp Thr Asp Ser Glu Glu Glu Ile Arg Glu Ala Phe Arg
305                 310                 315                 320

Val Phe Asp Lys Asp Gly Asn Gly Tyr Ile Ser Ala Ala Gln Leu Arg
                325                 330                 335
His Val Met Thr Asn Leu Gly Glu Lys Leu Thr Asp Glu Glu Val Asp
            340                 345                 350
Glu Met Ile Arg Glu Ala Asp Ile Asp Gly Asp Gly Gln Val Asn Tyr
        355                 360                 365
Glu Glu Phe Val Gln Met Met Thr Ala Lys Gly Gly Lys Arg Arg Trp
    370                 375                 380
Lys Lys Asn Phe Ile Ala Val Ser Ala Ala Asn Arg Phe Lys Lys Ile
385                 390                 395                 400
Ser Ser Ser Gly Ala Leu Glu Leu Met Val Ser Lys Gly Glu Glu Leu
                405                 410                 415
Phe Thr Gly Val Val Pro Ile Leu Val Glu Leu Asp Gly Asp Val Asn
            420                 425                 430
Gly His Lys Phe Ser Val Ser Gly Glu Gly Glu Gly Asp Ala Thr Tyr
        435                 440                 445
Gly Lys Leu Thr Leu Lys Leu Ile Cys Thr Thr Gly Lys Leu Pro Val
    450                 455                 460
Pro Trp Pro Thr Leu Val Thr Thr Leu Gly Tyr Gly Leu Gln Cys Phe
465                 470                 475                 480
Ala Arg Tyr Pro Asp His Met Lys Gln His Asp Phe Phe Lys Ser Ala
                485                 490                 495
Met Pro Glu Gly Tyr Val Gln Glu Arg Thr Ile Phe Phe Lys Asp Asp
            500                 505                 510
Gly Asn Tyr Lys Thr Arg Ala Glu Val Lys Phe Glu Gly Asp Thr Leu
        515                 520                 525
Val Asn Arg Ile Glu Leu Lys Gly Ile Asp Phe Lys Glu Asp Gly Asn

    530                 535                 540

Ile Leu Gly His Lys Leu Glu Tyr Asn Tyr Asn Ser His Asn Val Tyr

545                 550                 555                 560

Ile Thr Ala Asp Lys Gln Lys Asn Gly Ile Lys Ala Asn Phe Lys Ile

                565                 570                 575

Arg His Asn Ile Glu Asp Gly Gly Val Gln Leu Ala Asp His Tyr Gln

            580                 585                 590

Gln Asn Thr Pro Ile Gly Asp Gly Pro Val Leu Leu Pro Asp Asn His

        595                 600                 605

Tyr Leu Ser Tyr Gln Ser Ala Leu Ser Lys Asp Pro Asn Glu Lys Arg

    610                 615                 620

Asp His Met Val Leu Leu Glu Phe Val Thr Ala Ala Gly Ile Thr Leu

625                 630                 635                 640

Gly Met Asp Glu Leu Tyr Lys

                645

<210> 42

<211> 653

<212> PRT

<213> Artificial Sequence

<400> 42

Met Val Ser Lys Gly Glu Glu Leu Phe Thr Gly Val Val Pro Ile Leu

1               5                   10                  15

Val Glu Leu Asp Gly Asp Val Asn Gly His Arg Phe Ser Val Ser Gly

            20                  25                  30

Glu Gly Glu Gly Asp Ala Thr Tyr Gly Lys Leu Thr Leu Lys Phe Ile

        35                  40                  45

Cys Thr Thr Gly Lys Leu Pro Val Pro Trp Pro Thr Leu Val Thr Thr

    50                  55                  60
Leu Thr Trp Gly Val Gln Cys Phe Ser Arg Tyr Pro Asp His Met Lys
65                  70                  75                  80
Gln His Asp Phe Phe Lys Ser Ala Met Pro Glu Gly Tyr Val Gln Glu
                85                  90                  95
Arg Thr Ile Phe Phe Lys Asp Asp Gly Asn Tyr Lys Thr Arg Ala Glu
            100                 105                 110
Val Lys Phe Glu Gly Asp Thr Leu Val Asn Arg Ile Glu Leu Lys Gly
        115                 120                 125
Ile Asp Phe Lys Glu Asp Gly Asn Ile Leu Gly His Lys Leu Glu Tyr
    130                 135                 140
Asn Tyr Ile Ser His Asn Val Tyr Ile Thr Ala Asp Lys Gln Lys Asn
145                 150                 155                 160
Gly Ile Lys Ala Asn Phe Lys Ile Arg His Asn Ile Glu Asp Gly Ser
                165                 170                 175
Val Gln Leu Ala Asp His Tyr Gln Gln Asn Thr Pro Ile Gly Asp Gly
            180                 185                 190
Pro Val Leu Leu Pro Asp Asn His Tyr Leu Ser Thr Gln Ser Ala Leu
        195                 200                 205
Ser Lys Asp Pro Lys Glu Lys Arg Asp His Met Val Leu Leu Glu Phe
    210                 215                 220
Val Thr Ala Ala Arg Met His Asp Gln Leu Thr Glu Glu Gln Ile Ala
225                 230                 235                 240
Glu Phe Lys Glu Ala Phe Ser Leu Phe Asp Lys Asp Gly Asp Gly Thr
                245                 250                 255
Ile Thr Thr Lys Glu Leu Gly Thr Val Met Arg Ser Leu Gly Gln Asn
            260                 265                 270

Pro Thr Glu Ala Glu Leu Gln Asp Met Ile Asn Glu Val Asp Ala Asp
        275                 280                 285
Gly Asn Gly Thr Ile Tyr Phe Pro Glu Phe Leu Thr Met Met Ala Arg
    290                 295                 300
Lys Met Lys Asp Thr Asp Ser Glu Glu Glu Ile Arg Glu Ala Phe Arg
305                 310                 315                 320
Val Phe Asp Lys Asp Gly Asn Gly Tyr Ile Ser Ala Ala Gln Leu Arg
                325                 330                 335
His Val Met Thr Asn Leu Gly Glu Lys Leu Thr Asp Glu Glu Val Asp
            340                 345                 350
Glu Met Ile Arg Glu Ala Asp Ile Asp Gly Asp Gly Gln Val Asn Tyr
        355                 360                 365
Glu Glu Phe Val Gln Met Met Thr Ala Lys Gly Gly Lys Arg Arg Trp
    370                 375                 380
Lys Lys Asn Phe Ile Ala Val Ser Ala Ala Asn Arg Phe Lys Lys Ile
385                 390                 395                 400
Ser Ser Ser Gly Ala Leu Glu Leu Met Thr Gly Lys Leu Pro Val Pro
                405                 410                 415
Trp Pro Thr Leu Val Thr Thr Leu Gly Tyr Gly Leu Gln Cys Phe Ala
            420                 425                 430
Arg Tyr Pro Asp His Met Lys Gln His Asp Phe Phe Lys Ser Ala Met
        435                 440                 445
Pro Glu Gly Tyr Val Gln Glu Arg Thr Ile Phe Phe Lys Asp Asp Gly
    450                 455                 460
Asn Tyr Lys Thr Arg Ala Glu Val Lys Phe Glu Gly Asp Thr Leu Val
465                 470                 475                 480
Asn Arg Ile Glu Leu Lys Gly Ile Asp Phe Lys Glu Asp Gly Asn Ile

                485                 490                 495
Leu Gly His Lys Leu Glu Tyr Asn Tyr Asn Ser His Asn Val Tyr Ile
            500                 505                 510
Thr Ala Asp Lys Gln Lys Asn Gly Ile Lys Ala Asn Phe Lys Ile Arg
        515                 520                 525
His Asn Ile Glu Asp Gly Gly Val Gln Leu Ala Asp His Tyr Gln Gln
    530                 535                 540
Asn Thr Pro Ile Gly Asp Gly Pro Val Leu Leu Pro Asp Asn His Tyr
545 550 550 560
Leu Ser Tyr Gln Ser Ala Leu Ser Lys Asp Pro Asn Glu Lys Arg Asp
                565                 570                 575
His Met Val Leu Leu Glu Phe Val Thr Ala Ala Gly Ile Thr Leu Gly
            580                 585                 590
Met Asp Glu Leu Tyr Lys Gly Gly Ser Gly Gly Met Val Ser Lys Gly
        595                 600                 605
Glu Glu Leu Phe Thr Gly Val Val Pro Ile Leu Val Glu Leu Asp Gly
    610                 615                 620
Asp Val Asn Gly His Lys Phe Ser Val Ser Gly Glu Gly Glu Gly Asp
625                 630                 635                 640
Ala Thr Tyr Gly Lys Leu Thr Leu Lys Leu Ile Cys Thr
                645                 650
<210> 43
<211> 653
<212> PRT
<213> Artificial Sequence
<400> 43
Met Val Ser Lys Gly Glu Glu Leu Phe Thr Gly Val Val Pro Ile Leu

1               5                   10                  15

Val Glu Leu Asp Gly Asp Val Asn Gly His Arg Phe Ser Val Ser Gly

            20                  25                  30

Glu Gly Glu Gly Asp Ala Thr Tyr Gly Lys Leu Thr Leu Lys Phe Ile

        35                  40                  45

Cys Thr Thr Gly Lys Leu Pro Val Pro Trp Pro Thr Leu Val Thr Thr

    50                  55                  60

Leu Thr Trp Gly Val Gln Cys Phe Ser Arg Tyr Pro Asp His Met Lys

65                  70                  75                  80

Gln His Asp Phe Phe Lys Ser Ala Met Pro Glu Gly Tyr Val Gln Glu

                85                  90                  95

Arg Thr Ile Phe Phe Lys Asp Asp Gly Asn Tyr Lys Thr Arg Ala Glu

            100                 105                 110

Val Lys Phe Glu Gly Asp Thr Leu Val Asn Arg Ile Glu Leu Lys Gly

        115                 120                 125

Ile Asp Phe Lys Glu Asp Gly Asn Ile Leu Gly His Lys Leu Glu Tyr

    130                 135                 140

Asn Tyr Ile Ser His Asn Val Tyr Ile Thr Ala Asp Lys Gln Lys Asn

145                 150                 155                 160

Gly Ile Lys Ala Asn Phe Lys Ile Arg His Asn Ile Glu Asp Gly Ser

                165                 170                 175

Val Gln Leu Ala Asp His Tyr Gln Gln Asn Thr Pro Ile Gly Asp Gly

            180                 185                 190

Pro Val Leu Leu Pro Asp Asn His Tyr Leu Ser Thr Gln Ser Ala Leu

        195                 200                 205

Ser Lys Asp Pro Lys Glu Lys Arg Asp His Met Val Leu Leu Glu Phe

    210                 215                 220

Val Thr Ala Ala Arg Met His Asp Gln Leu Thr Glu Glu Gln Ile Ala
225                 230                 235                 240
Glu Phe Lys Glu Ala Phe Ser Leu Phe Asp Lys Asp Gly Asp Gly Thr
                245                 250                 255
Ile Thr Thr Lys Glu Leu Gly Thr Val Met Arg Ser Leu Gly Gln Asn
            260                 265                 270
Pro Thr Glu Ala Glu Leu Gln Asp Met Ile Asn Glu Val Asp Ala Asp
        275                 280                 285
Gly Asn Gly Thr Ile Tyr Phe Pro Glu Phe Leu Thr Met Met Ala Arg
    290                 295                 300
Lys Met Lys Asp Thr Asp Ser Glu Glu Glu Ile Arg Glu Ala Phe Arg
305                 310                 315                 320
Val Phe Asp Lys Asp Gly Asn Gly Tyr Ile Ser Ala Ala Gln Leu Arg
                325                 330                 335
His Val Met Thr Asn Leu Gly Glu Lys Leu Thr Asp Glu Glu Val Asp
            340                 345                 350
Glu Met Ile Arg Glu Ala Asp Ile Asp Gly Asp Gly Gln Val Asn Tyr
        355                 360                 365
Glu Glu Phe Val Gln Met Met Thr Ala Lys Gly Gly Lys Arg Arg Trp
    370                 375                 380
Lys Lys Asn Phe Ile Ala Val Ser Ala Ala Asn Arg Phe Lys Lys Ile
385                 390                 395                 400
Ser Ser Ser Gly Ala Leu Glu Leu Met Gln Lys Asn Gly Ile Lys Ala
                405                 410                 415
Asn Phe Lys Ile Arg His Asn Ile Glu Asp Gly Gly Val Gln Leu Ala
            420                 425                 430
Asp His Tyr Gln Gln Asn Thr Pro Ile Gly Asp Gly Pro Val Leu Leu

        435                 440                 445
Pro Asp Asn His Tyr Leu Ser Tyr Gln Ser Ala Leu Ser Lys Asp Pro
    450                 455                 460
Asn Glu Lys Arg Asp His Met Val Leu Leu Glu Phe Val Thr Ala Ala
465                 470                 475                 480
Gly Ile Thr Leu Gly Met Asp Glu Leu Tyr Lys Gly Gly Ser Gly Gly
                485                 490                 495
Met Val Ser Lys Gly Glu Glu Leu Phe Thr Gly Val Val Pro Ile Leu
            500                 505                 510
Val Glu Leu Asp Gly Asp Val Asn Gly His Lys Phe Ser Val Ser Gly
        515                 520                 525
Glu Gly Glu Gly Asp Ala Thr Tyr Gly Lys Leu Thr Leu Lys Leu Ile
    530                 535                 540
Cys Thr Thr Gly Lys Leu Pro Val Pro Trp Pro Thr Leu Val Thr Thr
545                 550                 555                 560
Leu Gly Tyr Gly Leu Gln Cys Phe Ala Arg Tyr Pro Asp His Met Lys
                565                 570                 575
Gln His Asp Phe Phe Lys Ser Ala Met Pro Glu Gly Tyr Val Gln Glu
            580                 585                 590
Arg Thr Ile Phe Phe Lys Asp Asp Gly Asn Tyr Lys Thr Arg Ala Glu
        595                 600                 605
Val Lys Phe Glu Gly Asp Thr Leu Val Asn Arg Ile Glu Leu Lys Gly
    610                 615                 620
Ile Asp Phe Lys Glu Asp Gly Asn Ile Leu Gly His Lys Leu Glu Tyr
625                 630                 635                 640
Asn Tyr Asn Ser His Asn Val Tyr Ile Thr Ala Asp Lys
                645                 650

<210> 44

<211> 653

<212> PRT

<213> Artificial Sequence

<400> 44

Met Val Ser Lys Gly Glu Glu Leu Phe Thr Gly Val Val Pro Ile Leu

1               5                   10                  15

Val Glu Leu Asp Gly Asp Val Asn Gly His Arg Phe Ser Val Ser Gly

            20                  25                  30

Glu Gly Glu Gly Asp Ala Thr Tyr Gly Lys Leu Thr Leu Lys Phe Ile

        35                  40                  45

Cys Thr Thr Gly Lys Leu Pro Val Pro Trp Pro Thr Leu Val Thr Thr

    50                  55                  60

Leu Thr Trp Gly Val Gln Cys Phe Ser Arg Tyr Pro Asp His Met Lys

65                  70                  75                  80

Gln His Asp Phe Phe Lys Ser Ala Met Pro Glu Gly Tyr Val Gln Glu

                85                  90                  95

Arg Thr Ile Phe Phe Lys Asp Asp Gly Asn Tyr Lys Thr Arg Ala Glu

            100                 105                 110

Val Lys Phe Glu Gly Asp Thr Leu Val Asn Arg Ile Glu Leu Lys Gly

        115                 120                 125

Ile Asp Phe Lys Glu Asp Gly Asn Ile Leu Gly His Lys Leu Glu Tyr

    130                 135                 140

Asn Tyr Ile Ser His Asn Val Tyr Ile Thr Ala Asp Lys Gln Lys Asn

145                 150                 155                 160

Gly Ile Lys Ala Asn Phe Lys Ile Arg His Asn Ile Glu Asp Gly Ser

                165                 170                 175

Val Gln Leu Ala Asp His Tyr Gln Gln Asn Thr Pro Ile Gly Asp Gly
            180                 185                 190
Pro Val Leu Leu Pro Asp Asn His Tyr Leu Ser Thr Gln Ser Ala Leu
        195                 200                 205
Ser Lys Asp Pro Lys Glu Lys Arg Asp His Met Val Leu Leu Glu Phe
    210                 215                 220
Val Thr Ala Ala Arg Met His Asp Gln Leu Thr Glu Glu Gln Ile Ala
225                 230                 235                 240
Glu Phe Lys Glu Ala Phe Ser Leu Phe Asp Lys Asp Gly Asp Gly Thr
                245                 250                 255
Ile Thr Thr Lys Glu Leu Gly Thr Val Met Arg Ser Leu Gly Gln Asn
            260                 265                 270
Pro Thr Glu Ala Glu Leu Gln Asp Met Ile Asn Glu Val Asp Ala Asp
        275                 280                 285
Gly Asn Gly Thr Ile Tyr Phe Pro Glu Phe Leu Thr Met Met Ala Arg
    290                 295                 300
Lys Met Lys Asp Thr Asp Ser Glu Glu Glu Ile Arg Glu Ala Phe Arg
305                 310                 315                 320
Val Phe Asp Lys Asp Gly Asn Gly Tyr Ile Ser Ala Ala Gln Leu Arg
                325                 330                 335
His Val Met Thr Asn Leu Gly Glu Lys Leu Thr Asp Glu Glu Val Asp
            340                 345                 350
Glu Met Ile Arg Glu Ala Asp Ile Asp Gly Asp Gly Gln Val Asn Tyr
        355                 360                 365
Glu Glu Phe Val Gln Met Met Thr Ala Lys Gly Gly Lys Arg Arg Trp
    370                 375                 380
Lys Lys Asn Phe Ile Ala Val Ser Ala Ala Asn Arg Phe Lys Lys Ile

385                 390                 395                 400

Ser Ser Ser Gly Ala Leu Glu Leu Met Asp Gly Gly Val Gln Leu Ala

                405                 410                 415

Asp His Tyr Gln Gln Asn Thr Pro Ile Gly Asp Gly Pro Val Leu Leu

            420                 425                 430

Pro Asp Asn His Tyr Leu Ser Tyr Gln Ser Ala Leu Ser Lys Asp Pro

        435                 440                 445

Asn Glu Lys Arg Asp His Met Val Leu Leu Glu Phe Val Thr Ala Ala

    450                 455                 460

Gly Ile Thr Leu Gly Met Asp Glu Leu Tyr Lys Gly Gly Ser Gly Gly

465                 470                 475                 480

Met Val Ser Lys Gly Glu Glu Leu Phe Thr Gly Val Val Pro Ile Leu

                485                 490                 495

Val Glu Leu Asp Gly Asp Val Asn Gly His Lys Phe Ser Val Ser Gly

            500                 505                 510

Glu Gly Glu Gly Asp Ala Thr Tyr Gly Lys Leu Thr Leu Lys Leu Ile

        515                 520                 525

Cys Thr Thr Gly Lys Leu Pro Val Pro Trp Pro Thr Leu Val Thr Thr

    530                 535                 540

Leu Gly Tyr Gly Leu Gln Cys Phe Ala Arg Tyr Pro Asp His Met Lys

545                 550                 555                 560

Gln His Asp Phe Phe Lys Ser Ala Met Pro Glu Gly Tyr Val Gln Glu

                565                 570                 575

Arg Thr Ile Phe Phe Lys Asp Asp Gly Asn Tyr Lys Thr Arg Ala Glu

            580                 585                 590

Val Lys Phe Glu Gly Asp Thr Leu Val Asn Arg Ile Glu Leu Lys Gly

        595                 600                 605

Ile Asp Phe Lys Glu Asp Gly Asn Ile Leu Gly His Lys Leu Glu Tyr
    610                 615                 620
Asn Tyr Asn Ser His Asn Val Tyr Ile Thr Ala Asp Lys Gln Lys Asn
625                 630                 635                 640
Gly Ile Lys Ala Asn Phe Lys Ile Arg His Asn Ile Glu
                645                 650
<210> 45
<211> 653
<212> PRT
<213> Artificial Sequence
<400> 45
Met Val Ser Lys Gly Glu Glu Leu Phe Thr Gly Val Val Pro Ile Leu
1               5                   10                  15
Val Glu Leu Asp Gly Asp Val Asn Gly His Arg Phe Ser Val Ser Gly
            20                  25                  30
Glu Gly Glu Gly Asp Ala Thr Tyr Gly Lys Leu Thr Leu Lys Phe Ile
        35                  40                  45
Cys Thr Thr Gly Lys Leu Pro Val Pro Trp Pro Thr Leu Val Thr Thr
    50                  55                  60
Leu Thr Trp Gly Val Gln Cys Phe Ser Arg Tyr Pro Asp His Met Lys
65                  70                  75                  80
Gln His Asp Phe Phe Lys Ser Ala Met Pro Glu Gly Tyr Val Gln Glu
                85                  90                  95
Arg Thr Ile Phe Phe Lys Asp Asp Gly Asn Tyr Lys Thr Arg Ala Glu
            100                 105                 110
Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140
Asn Tyr Ile Ser His Asn Val Tyr Ile Thr Ala Asp Lys Gln Lys Asn
145                 150                 155                 160
Gly Ile Lys Ala Asn Phe Lys Ile Arg His Asn Ile Glu Asp Gly Ser
                165                 170                 175
Val Gln Leu Ala Asp His Tyr Gln Gln Asn Thr Pro Ile Gly Asp Gly
            180                 185                 190
Pro Val Leu Leu Pro Asp Asn His Tyr Leu Ser Thr Gln Ser Ala Leu
        195                 200                 205
Ser Lys Asp Pro Lys Glu Lys Arg Asp His Met Val Leu Leu Glu Phe
    210                 215                 220
Val Thr Ala Ala Arg Met His Asp Gln Leu Thr Glu Glu Gln Ile Ala
225                 230                 235                 240
Glu Phe Lys Glu Ala Phe Ser Leu Phe Asp Lys Asp Gly Asp Gly Thr
                245                 250                 255
Ile Thr Thr Lys Glu Leu Gly Thr Val Met Arg Ser Leu Gly Gln Asn
            260                 265                 270
Pro Thr Glu Ala Glu Leu Gln Asp Met Ile Asn Glu Val Asp Ala Asp
        275                 280                 285
Gly Asn Gly Thr Ile Tyr Phe Pro Glu Phe Leu Thr Met Met Ala Arg
    290                 295                 300
Lys Met Lys Asp Thr Asp Ser Glu Glu Glu Ile Arg Glu Ala Phe Arg
305                 310                 315                 320
Val Phe Asp Lys Asp Gly Asn Gly Tyr Ile Ser Ala Ala Gln Leu Arg
                325                 330                 335
His Val Met Thr Asn Leu Gly Glu Lys Leu Thr Asp Glu Glu Val Asp

            340                 345                 350
Glu Met Ile Arg Glu Ala Asp Ile Asp Gly Asp Gly Gln Val Asn Tyr
        355                 360                 365
Glu Glu Phe Val Gln Met Met Thr Ala Lys Gly Gly Lys Arg Arg Trp
    370                 375                 380
Lys Lys Asn Phe Ile Ala Val Ser Ala Ala Asn Arg Phe Lys Lys Ile
385                 390                 395                 400
Ser Ser Ser Gly Ala Leu Glu Leu Met Leu Pro Asp Asn His Tyr Leu
                405                 410                 415
Ser Tyr Gln Ser Ala Leu Ser Lys Asp Pro Asn Glu Lys Arg Asp His
            420                 425                 430
Met Val Leu Leu Glu Phe Val Thr Ala Ala Gly Ile Thr Leu Gly Met
        435                 440                 445
Asp Glu Leu Tyr Lys Gly Gly Ser Gly Gly Met Val Ser Lys Gly Glu
    450                 455                 460
Glu Leu Phe Thr Gly Val Val Pro Ile Leu Val Glu Leu Asp Gly Asp
465                 470                 475                 480
Val Asn Gly His Lys Phe Ser Val Ser Gly Glu Gly Glu Gly Asp Ala
                485                 490                 495
Thr Tyr Gly Lys Leu Thr Leu Lys Leu Ile Cys Thr Thr Gly Lys Leu
            500                 505                 510
Pro Val Pro Trp Pro Thr Leu Val Thr Thr Leu Gly Tyr Gly Leu Gln
        515                 520                 525
Cys Phe Ala Arg Tyr Pro Asp His Met Lys Gln His Asp Phe Phe Lys
    530                 535                 540
Ser Ala Met Pro Glu Gly Tyr Val Gln Glu Arg Thr Ile Phe Phe Lys
545                 550                 555                 560

Asp Asp Gly Asn Tyr Lys Thr Arg Ala Glu Val Lys Phe Glu Gly Asp
                565                 570                 575
Thr Leu Val Asn Arg Ile Glu Leu Lys Gly Ile Asp Phe Lys Glu Asp
            580                 585                 590
Gly Asn Ile Leu Gly His Lys Leu Glu Tyr Asn Tyr Asn Ser His Asn
        595                 600                 605
Val Tyr Ile Thr Ala Asp Lys Gln Lys Asn Gly Ile Lys Ala Asn Phe
    610                 615                 620
Lys Ile Arg His Asn Ile Glu Asp Gly Gly Val Gln Leu Ala Asp His
625                 630                 635                 640
Tyr Gln Gln Asn Thr Pro Ile Gly Asp Gly Pro Val Leu
                645                 650
<210> 46
<211> 653
<212> PRT
<213> Artificial Sequence
<400> 46
Met Val Ser Lys Gly Glu Glu Leu Phe Thr Gly Val Val Pro Ile Leu
1               5                   10                  15
Val Glu Leu Asp Gly Asp Val Asn Gly His Arg Phe Ser Val Ser Gly
            20                  25                  30
Glu Gly Glu Gly Asp Ala Thr Tyr Gly Lys Leu Thr Leu Lys Phe Ile
        35                  40                  45
Cys Thr Thr Gly Lys Leu Pro Val Pro Trp Pro Thr Leu Val Thr Thr
    50                  55                  60
Leu Thr Trp Gly Val Gln Cys Phe Ser Arg Tyr Pro Asp His Met Lys
65                  70                  75                  80

Gln His Asp Phe Phe Lys Ser Ala Met Pro Glu Gly Tyr Val Gln Glu
                85                  90                  95
Arg Thr Ile Phe Phe Lys Asp Asp Gly Asn Tyr Lys Thr Arg Ala Glu
            100                 105                 110
Val Lys Phe Glu Gly Asp Thr Leu Val Asn Arg Ile Glu Leu Lys Gly
        115                 120                 125
Ile Asp Phe Lys Glu Asp Gly Asn Ile Leu Gly His Lys Leu Glu Tyr
    130                 135                 140
Asn Tyr Ile Ser His Asn Val Tyr Ile Thr Ala Asp Lys Gln Lys Asn
145                 150                 155                 160
Gly Ile Lys Ala Asn Phe Lys Ile Arg His Asn Ile Glu Asp Gly Ser
                165                 170                 175
Val Gln Leu Ala Asp His Tyr Gln Gln Asn Thr Pro Ile Gly Asp Gly
            180                 185                 190
Pro Val Leu Leu Pro Asp Asn His Tyr Leu Ser Thr Gln Ser Ala Leu
        195                 200                 205
Ser Lys Asp Pro Lys Glu Lys Arg Asp His Met Val Leu Leu Glu Phe
    210                 215                 220
Val Thr Ala Ala Arg Met His Asp Gln Leu Thr Glu Glu Gln Ile Ala
225                 230                 235                 240
Glu Phe Lys Glu Ala Phe Ser Leu Phe Asp Lys Asp Gly Asp Gly Thr
                245                 250                 255
Ile Thr Thr Lys Glu Leu Gly Thr Val Met Arg Ser Leu Gly Gln Asn
            260                 265                 270
Pro Thr Glu Ala Glu Leu Gln Asp Met Ile Asn Glu Val Asp Ala Asp
        275                 280                 285
Gly Asn Gly Thr Ile Tyr Phe Pro Glu Phe Leu Thr Met Met Ala Arg

    290                 295                 300
Lys Met Lys Asp Thr Asp Ser Glu Glu Glu Ile Arg Glu Ala Phe Arg
305                 310                 315                 320
Val Phe Asp Lys Asp Gly Asn Gly Tyr Ile Ser Ala Ala Gln Leu Arg
                325                 330                 335
His Val Met Thr Asn Leu Gly Glu Lys Leu Thr Asp Glu Glu Val Asp
            340                 345                 350
Glu Met Ile Arg Glu Ala Asp Ile Asp Gly Asp Gly Gln Val Asn Tyr
        355                 360                 365
Glu Glu Phe Val Gln Met Met Thr Ala Lys Gly Gly Lys Arg Arg Trp
    370                 375                 380
Lys Lys Asn Phe Ile Ala Val Ser Ala Ala Asn Arg Phe Lys Lys Ile
385                 390                 395                 400
Ser Ser Ser Gly Ala Leu Glu Leu Met Ile Thr Leu Gly Met Asp Glu
                405                 410                 415
Leu Tyr Lys Gly Gly Ser Gly Gly Met Val Ser Lys Gly Glu Glu Leu
            420                 425                 430
Phe Thr Gly Val Val Pro Ile Leu Val Glu Leu Asp Gly Asp Val Asn
        435                 440                 445
Gly His Lys Phe Ser Val Ser Gly Glu Gly Glu Gly Asp Ala Thr Tyr
    450                 455                 460
Gly Lys Leu Thr Leu Lys Leu Ile Cys Thr Thr Gly Lys Leu Pro Val
465                 470                 475                 480
Pro Trp Pro Thr Leu Val Thr Thr Leu Gly Tyr Gly Leu Gln Cys Phe
                485                 490                 495
Ala Arg Tyr Pro Asp His Met Lys Gln His Asp Phe Phe Lys Ser Ala
            500                 505                 510

Met Pro Glu Gly Tyr Val Gln Glu Arg Thr Ile Phe Phe Lys Asp Asp
        515                 520                 525
Gly Asn Tyr Lys Thr Arg Ala Glu Val Lys Phe Glu Gly Asp Thr Leu
    530                 535                 540
Val Asn Arg Ile Glu Leu Lys Gly Ile Asp Phe Lys Glu Asp Gly Asn
545 550 550 560
Ile Leu Gly His Lys Leu Glu Tyr Asn Tyr Asn Ser His Asn Val Tyr
                565                 570                 575
Ile Thr Ala Asp Lys Gln Lys Asn Gly Ile Lys Ala Asn Phe Lys Ile
            580                 585                 590
Arg His Asn Ile Glu Asp Gly Gly Val Gln Leu Ala Asp His Tyr Gln
        595                 600                 605
Gln Asn Thr Pro Ile Gly Asp Gly Pro Val Leu Leu Pro Asp Asn His
    610                 615                 620
Tyr Leu Ser Tyr Gln Ser Ala Leu Ser Lys Asp Pro Asn Glu Lys Arg
625                 630                 635                 640
Asp His Met Val Leu Leu Glu Phe Val Thr Ala Ala Gly
                645                 650

## Description

### TECHNICAL FIELD

The present invention relates to a fluorescent indicator used for analyzing intermolecular interaction using fluorescence resonance energy transfer (FRET), and a use thereof. More specifically, the present invention relates to a fluorescent indicator prepared by binding two fluorescent molecules via a target sequence, and a method for analyzing intermolecular interaction using the above fluorescent indicator.

### BACKGROUND ART

Cameleons are genetically-encoded fluorescent indicators for Ca²⁺ based on green fluorescent protein (GFP) variants and calmodulin (CaM) (Miyawaki A. et al., (1997) Nature 388, 882-887; and Tsien, R. Y. (1998) Ann. Rev. Biochem. 67, 509-544). Cameleons are chimeric proteins composed of a short-wavelength variant of GFP, calmodulin (CaM), a glycylglycine linker, the CaM-binding peptide of myosin light-chain kinase (M13), and a long-wavelength variant of GFP. Ca²⁺ binding to CaM initiates an intermolecular interaction between CaM and M13, which changes the chimeric protein from an extended to a more compact conformation, thereby increasing the efficiency of FRET from the shorter- to the longer-wavelength variant of GFP. Yellow cameleons (YCs) have cyan fluorescent protein (CFP) and yellow fluorescent protein (YFP) as the FRET donor and acceptor, respectively. Yellow cameleons (YCs) are classified into several groups based on the composition of their Ca²⁺ -sensing domains. For example, YC2 has an intact CaM, and thus shows high affinity for Ca²⁺. On the other hand, YC3 and YC4 are low-affinity indicators due to mutations in the Ca²⁺ binding loops of their CaM domains. These YCs have been made more resistant to acidification by replacing the original YFP with EYFP.1 (Miyawaki A. et al., (1999) Proc. Natl. Acad. Sci. USA 96, 2135-2140). The improved YCs include YC2.1 and YC3.1. In addition, some YCs have been made to mature more quickly by using especially bright versions of YFP such as citrine (Griesbeck O. et al., (2001) J. Biol. Chem. 276, 29188-29194) or Venus (Nagai T. et al., (2002) Nat. Biotechnol. 20, 87-90). As mentioned above, YCs have been improved mainly by optimizing the YFP component.

Despite the above-mentioned improvements, YCs still suffer from poor dynamic range. The best versions available currently, such as YC2.12 or YC3.12, exhibit at most a 120% change in the ratio of YFP/CFP upon Ca²⁺ binding *in vitro.*

These YCs do not have good signal-to-noise ratios, particularly when they are targeted to organelles or submicroscopic environments, due to low levels of signal. It has been also suggested that their dynamic range is attenuated *in vivo* depending on the abundance of endogenous CaM and CaM-binding proteins that may interact with the sensing domains of YCs.

### DISCLOSURE OF THE INVENTION

It is an object of the present invention to provide a novel fluorescent indicator used for analyzing intermolecular interaction or a change in the intramolecular structure, using fluorescence resonance energy transfer (FRET). It is another object of the present invention to provide a fluorescent indicator exhibiting a high dynamic range.

The present inventors have conducted intensive studies directed towards achieving the aforementioned objects, and they have attempted to modify the acceptor in order to increase the dynamic range of the indicator. To achieve a Ca²⁺-dependent, large change in the relative orientation and distance between the fluorophores of CFP and YFP, they assumed that optimization of the length and sequence of the linkers used in YCs would yield only moderate improvement. Thus, they took an approach that used circularly permutated GFPs (cpGFP), in which the amino and carboxyl portions were interchanged and reconnected by a short spacer between the original termini (Baird G. S. et al., (1999) Proc. Natl. Acad. Sci. USA *96,* 11241-11246; and Topell, S. et al., (1999) FEBS Lett. *457*, 283-289). By using cpYFPs that are resistant to acidification and that mature efficiently, the present inventors attempted to vary the relative orientation of the two chromophores' transition dipoles. As a result, they have found that a fluorescent indicator exhibiting an excellent dynamic range can be obtained. The present invention has been completed based on these findings.

That is to say, the present invention provides: a fluorescent indicator, which has a structure such that a donor fluorescent protein and an acceptor fluorescent protein bind to both termini of the target sequence of an analytical substance, wherein the analytical substance binds to or acts on said target sequence, so that the three-dimensional structure of the indicator can be changed, thereby generating fluorescence resonance energy transfer (FRET), said fluorescent indicator being characterized in that the said donor fluorescent protein and/or said acceptor fluorescent protein are circularly permuted fluorescent proteins obtained by substituting the amino acid sequence on the N-terminal side of a wild-type fluorescent protein or a mutant protein thereof with the amino acid sequence on the C-terminal side thereof, and in that the thus obtained fluorescent proteins have a fluorescent peak wavelength substantially identical to that of a florescent protein, which has not yet been subjected to the circular permutation.

Preferably, the fluorescent protein is GFP, CFP, YFP, RFP, BFP, or a mutant thereof. Preferably, the donor fluorescent protein is CFP or a mutant thereof, and the acceptor fluorescent protein is YFP or a mutant thereof.

Preferably, the donor fluorescent protein and/or the acceptor fluorescent protein are circularly permuted fluorescent proteins, which are obtained by substituting the amino acid sequence on the N-terminal side with the amino acid sequence on the C-terminal side with respect to amino acid residues positioned in a β turn in the amino acid sequence of a wild-type fluorescent protein or a mutant protein thereof. Preferably, the amino acid residues positioned in the β turn are amino acid residues, which are positioned such that the dynamic range of the fluorescence of a fluorescent protein can be increased.

Preferably, the acceptor fluorescent protein is a circularly permuted version of the fluorescent protein Venus. Preferably, the circularly permuted versions of Venus are cp49Venus, cp157Venus, cp173Venus, cp195Venus, or cp229Venus.

Preferably, the present invention provides a fluorescent indicator, which further comprises a target peptide component and a linker component,
wherein the target sequence of the analytical substance further comprises a peptide-binding domain for allowing the target peptide component to bind thereto,
wherein the linker component allows the target sequence of the analytical substance to covalently bind to the target peptide component, and the target sequence and the target peptide component covalently bind to either the acceptor fluorescent molecular component or the donor fluorescent molecular component, and
wherein the analytical substance binding to the target sequence induces a change in the relative positions or orientations of the target peptide component and the peptide-binding domain, and the relative positions or orientations of the donor molecular component and acceptor molecular component are then changed, thereby changing the efficiency of fluorescence resonance energy transfer (FRET).

Preferably, the target sequence is calmodulin, cGMP-dependent protein kinase, a steroid hormone receptor, a ligand-binding domain of a steroid hormone receptor, protein kinase C, inositol-1,4,5-triphosphate receptor, or recobelin. It is particularly preferably calmodulin.

Preferably, the target peptide component is skeletal muscle myosin light chain kinase (skMLCKp), smooth muscle light chain kinase (smMLCK), calmodulin kinase II (CaMKII), caldesmon, calspermine, phosphofructokinase, calcineurin, phosphorylase kinase, Ca²⁺-ATPase, 59 Kda phosphodiesterase (PDE), 60 Kda phosphodiesterase (PDE), nitric oxide synthase, type I adenylyl cyclase, *Bordetella pertussis* adenylyl cyclase, neuromodulin, spectrin, myristoylated alanine-rich C kinase substrate (MARCKS), MacMARCKS(F52), β-Adducin, heat shock protein HSP90a, human immunodeficiency virus envelope glycoprotein 160 (HIV-1 gp160), brush-boarder myosin heavy chain-I (BBMHBI), dilute myosin heavy chain (MHC), mastoparan, melittin, glucagon, secretin, vasoactive intestinal peptide (VIP), gastrin inhibitory peptide (GIP), or a calmodulin-binding domain of calmodulin-binding peptide-2 (Model peptide CBP2).

Preferably, the linker component is a peptide component consisting of 1 to 30 amino acid residues.

The fluorescent indicator of the present invention preferably further comprises a localizing sequence. Preferably, the localizing sequence is a nucleus-localizing sequence, an endoplasmic reticulum-localizing sequence, a peroxisome-localizing sequence, a mitochondrion-localizing sequence, a Goldi apparatus-localizing sequence, or a cell membrane-localizing sequence.

Particularly preferably, the present invention provides a fluorescent indicator having the amino acid sequence shown in any one of SEQ ID NOS: 42, 43, 44, 45, and 46.

In another aspect, the present invention provides a method for detecting or measuring an analytical substance contained in a sample, which comprises:
(1) a step of allowing a sample to come into contact with the fluorescent indicator of the present invention;
(2) a step of exciting a donor component; and
(3) a step of measuring the level of fluorescence resonance energy transfer in the sample that corresponds to the concentration or activity of the analytical substance contained in the sample.

Preferably, the sample is a living cell, and the above contact step includes incorporation of the fluorescent indicator into such a living cell. Preferably, the step of incorporating the fluorescent indicator into a cell comprises transfection of the cell with an expression vector which contains an expression regulatory sequence that is functionally ligated to a nucleic acid sequence encoding the expression of the fluorescent indicator.

In a further aspect, the present invention provides nucleic acid encoding the fluorescent indicator of the present invention, an expression vector containing the above nucleic acid, and a transformant having the above nucleic acid or expression vector.

### BRIEF DESCRIPTION OF THE DRAWINGS

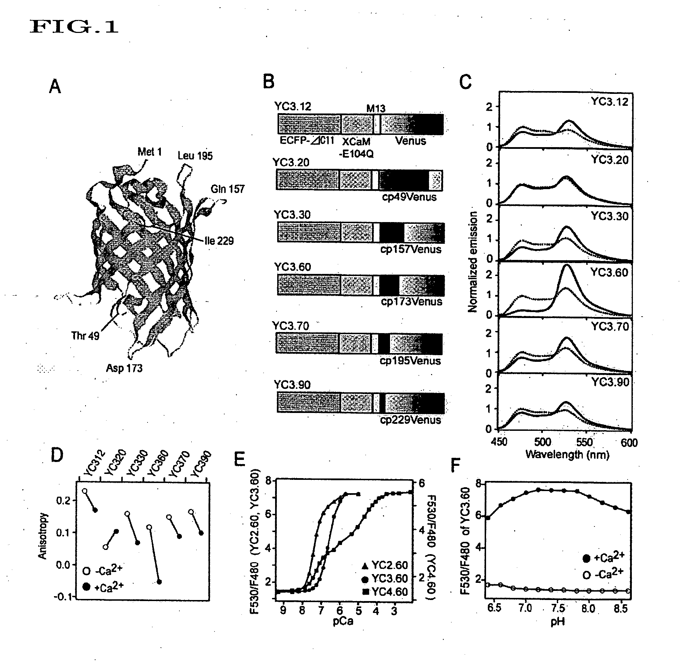

Figure 1 shows schematic structures and spectral properties of YC3.12 and the new YC variants. Figure 1A shows the three-dimensional structure of GFP with the positions of the original (Met1) and new N-termini (Thr49, Gln157, Asp173, Leu195, and Ile229). Figure 1B shows domain structures of YC3.12 (SEQ ID NO: 41), YC3.20 (SEQ ID NO: 42), YC3.30 (SEQ ID NO: 43), YC3.60 (SEQ ID NO: 44), YC3.70 (SEQ ID NO: 45), and YC3.90 (SEQ ID NO: 46). XCaM represents *Xenopus* calmodulin. E104Q represents mutation of the conserved bidentate glutamate (E 104) at position 12 of the third Ca²⁺ binding loop to glutamine. Figure 1C shows emission spectra of YC variants (excitation at 435 nm) at zero (dotted line) and saturated Ca²⁺ (solid line). Figure 1D shows fluorescence anisotropy of YC variants (YC3.12, YC3.20, YC3.30, YC3.60, YC3.70, and YC3.90) at zero and saturated Ca²⁺. Figure 1E shows Ca²⁺ titration curves of YC2.60 (triangles), YC3.60 (circles) and YC4.60 (squares) at pH 7.4. Figure 1F shows pH titration curves of YC3.60 at zero and saturated Ca²⁺.

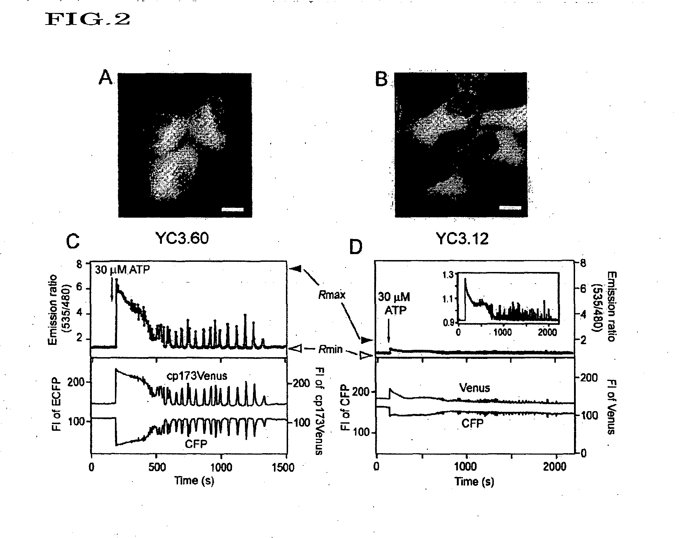

Figure 2 shows comparative measurements of Ca²⁺ dynamics in HeLa cells expressing YC3.60 and YC3.12. Figures 2A and 2B show fluorescence images (490 nm excitation, 535 nm emission) obtained using YC3.60 (A) and YC3.12 (B). Scale bar represents 10 µm. Figures 2C and 2D show Ca²⁺ transients reported by YC3.60 (C) and YC3.12 (D) in HeLa cells induced with 30 µMATP. Top: changes in emission ratios (535/480 nm) with *R*_{*max*} and *R*ₘᵢₙ values (indicated by solid and open arrowheads, respectively). Bottom: changes in fluorescence intensities of CFP and cp 173 Venus (C), and CFP and Venus (D). The sampling interval was 5 seconds.

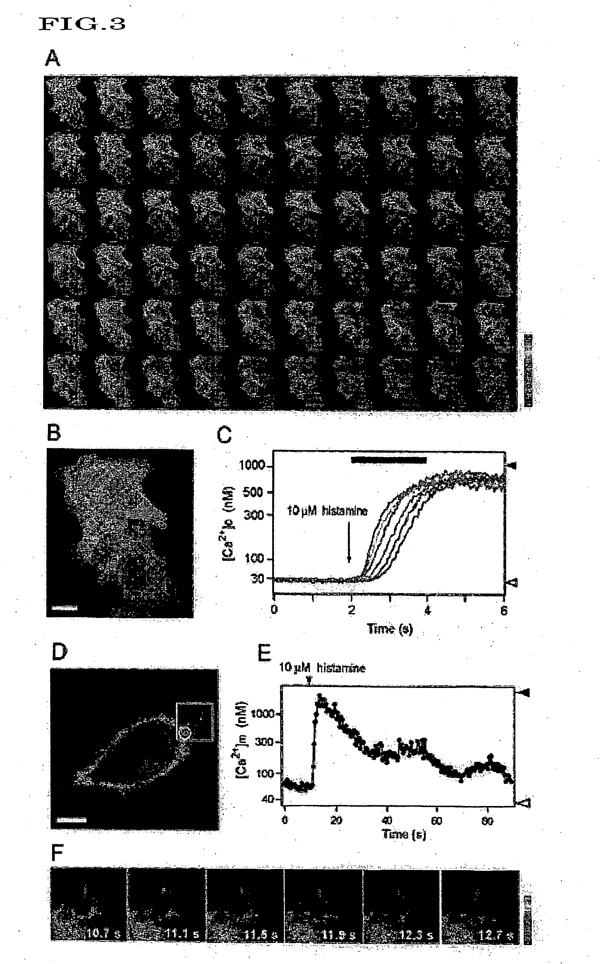

Figure 3 shows confocal imaging of [Ca²⁺]_{c} and [Ca²⁺]ₚₘ in HeLa cells using YC3.60. Figure 3A shows a series of confocal pseudo-colored ratio images showing propagation of [C²⁺]_{c}. These images were taken at video rate. Figure 3B shows a real-color image of the HeLa cells. In the top cell, 6 ROIs were placed for measuring the propagation speed. Scale bar represents 10 µm. Figure 3C shows time courses of changes in [Ca²⁺]_{c} in the 6 ROIs indicated in (B). *R*ₘₐₓ and *R*_{*min*} are indicated by a solid and an open arrowheads, respectively. The left-hand ordinate calibrates [Ca²⁺]_{c} in nM. A black horizontal bar indicates the time, during which the ratio images are shown in (A). Figure 3D shows a real-color image of a HeLa cell expressing YC3.60ₚₘ. Scale bar represents 5 µm. Figure 3E shows the histamine-induced change in [Ca²⁺]ₚₘ in the peripheral region indicated by an circle in (D). *R*ₘₐₓ and *R*ₘᵢₙ are indicated by a solid and an open arrowheads, respectively. The left-hand ordinate calibrates [Ca²⁺]ₚₘ in nM. Figure 3F shows a series of confocal pseudo-colored ratio images showing changes in [Ca²⁺]ₚₘ in filopodial structures.

### BEST MODE FOR CARRYING OUT THE INVENTION

As stated above, Cameleons and Yellow Cameleons (YCs) have been expected to work for investigating ensemble activity of neural circuitry in living animals. While the original and improved YCs display robust Ca²⁺ responses *in vitro* and in transiently transfected cell samples, their dynamic range is significantly reduced *in vivo* in the nervous systems of transgenic animals. In particular, no reliable Ca²⁺ measurements have been achieved in the brain of transgenic mice. Compared to the latest improved version of YC (YC3.12), YC3.60 is equally bright, but shows 5 to 6-fold larger dynamic range. Thus, YC3.60 gives a greatly enhanced signal-to-noise ratio, thereby enabling Ca²⁺ imaging experiments that were not possible with conventional YCs. For example, YC3.60 was targeted to the plasma membrane of HeLa cells, and thus [Ca²⁺]_{c} changes beneath the membrane of filopodial structures can be measured.

Baird *et al.,* who first reported the construction of a cpGFP, attempted to improve the dynamic range of YCs by replacing the donor CFP with a cpCFP, which has a new N-terminus at Tyr145 (Baird G. S. et al., (1999) Proc. Natl. Acad. Sci. USA 96, 11241-11246). However, in such cpCFP, the Ca²⁺-dependent emission ratio change was decreased to 15%. The present inventors improved upon this approach by testing multiple cpYFPs as the acceptor. cp49Venus, cp157Venus, cp173Venus, cp195Venus, and cp229Venus were generated from Venus, a bright version of YFP. All the five cpVenus proteins matured efficiently, probably because they all include F46L, the mutation that facilitates greatly the oxidation reaction for chromophore synthesis, and because their N-termini occur at surface-exposed loop regions of the β-barrel. In fact, rate of fluorescence development of cpGFP depends on the position of the new N and C termini (Topell S. et al., (1999) FEBS Lett. 457, 283-289). An increasing number of fluorescent indicators have been developed based on FRET between CFP and YFP (Miyawaki A. (2003) Dev. Cell 4, 295-305), in which the relative position between the two chromophores of CFP and YFP is varied. Thus, the cpVenus to be used in combination with CFP can be optimized for each specific application. Also, its combined use with cpCFPs can increase further the variation of the relative position of the two transition dipoles between donor and acceptor. Since cpGFP-based indicators for Ca²⁺ were developed a few years ago (Nakai J. et al., (2001) Nat. Biotechnol. 19, 137-141; and Nagai T. et al., (2001) Proc. Natl. Acad. Sci. USA 98, 3197-3202), cpGFPs themselves have been expected to become powerful tools comparable to pairs of GFP variants for FRET. The mode for carrying out the present invention is further described in detail below.

The fluorescent indicator of the present invention is a fluorescent, which has a structure such that a donor fluorescent protein and an acceptor fluorescent protein bind to both termini of the target sequence of an analytical substance, wherein the analytical substance binds to or acts on said target sequence, so that the three-dimensional structure of the indicator can be changed, thereby generating fluorescence resonance energy transfer (FRET), said fluorescent indicator being characterized in that the said donor fluorescent protein and/or said acceptor fluorescent protein are circularly permuted fluorescent proteins obtained by substituting the amino acid sequence on the N-terminal side of a wild-type fluorescent protein or a mutant protein thereof with the amino acid sequence on the C-terminal side thereof, and in that the thus obtained fluorescent proteins have a fluorescent peak wavelength substantially identical to that of a florescent protein, which has not yet been subjected to the circular permutation.

In the present invention, one type of protein acting as a donor protein in FRET and one type of protein acting as an acceptor protein in FRET are used. That is to say, in the present invention, two types of fluorescent proteins having different fluorescent wavelengths are used, and a fluorescence, which is generated as a result of the fluorescence resonance energy transfer occurring between such fluorescent proteins, is measured. The type of a fluorescent protein used in the present invention is not particularly limited. Examples may include a cyan fluorescent protein (CFP), a yellow fluorescent protein (YFP), a green fluorescent protein (GFP), a red fluorescent protein (RFP), a blue fluorescent protein (BFP), and a mutant thereof.

In the present specification, the expression "a cyan fluorescent protein, a yellow fluorescent protein, a green fluorescent protein, a red fluorescent protein, a blue fluorescent protein, or a mutant thereof" does not only mean known various fluorescent proteins, but also means that all the mutants thereof (for example, ECFP, EYFP, EGFP, ERFP, EBFP, etc., which are prepared by enhancing the fluorescence intensity of the above fluorescent proteins) are included. For example, a green fluorescent protein gene has been isolated and sequenced (Prasher D.C. et al., "Primary structure of the *Aequorea victoria* green fluorescent protein," Gene 111: 229-233, (1992)). The amino acid sequences of many other fluorescent proteins and mutants thereof have also been reported. Such proteins and mutants are described in Roger Y Tsien, Annu. Rev. Biochem. 1998. 67: 509-44, and in the cited publications thereof, for example. As such a green fluorescent protein (GFP), a yellow fluorescent protein (YFP), or a mutant thereof, those derived from *Aequorea aequorea* (e.g. *Aequorea victoria)* can be used, for example.

Several examples of GFP, YFP and a mutant thereof, are given below. However, examples are not limited thereto. It is to be noted that the expression "F99S" indicates that the amino acid residue F at position 99 is substituted with S. Substitutions of other amino acids are shown in the same above manner.
Wild type GFP;
GFP having amino acid mutations, F99S, M153T, and V163A;
GFP having amino acid mutation S65T;
GFP having amino acid mutations, F64L and S65T;
GFP having amino acid mutations, S65T, S72A, N149K, M153T, and I167T;
GFP having amino acid mutations, S202F and T203I;
GFP having amino acid mutations, T203I, S72A, and Y145F;
GFP having amino acid mutations, S65G, S72A, and T203F (YFP);
GFP having amino acid mutations, S65G, S72A, and T203H (YFP);
GFP having amino acid mutations, S65G, V68L, Q69K, S72A, and T203Y (EYFP-V68L, Q69K);
GFP having amino acid mutations, S65G, S72A, and T203Y (EYFP); and
GFP having amino acid mutations, S65G, S72A, K79R, and T203Y (YFP).

The nucleotide sequences of genes encoding the fluorescent proteins used in the present invention have been known. As such a gene encoding the fluorescent protein, a commercially available product can be used. For example, an EGFP vector, an EYFP vector, an ECFP vector, an EBFP vector, which are commercially available from Clontech, can be used.

In the present invention, GFP mutants, such as CFP, YFP, REP or a mutant thereof, are preferably used. For example, Venus that is a YFP mutant can be used. Please refer to Nagai T. et al., (2002) Nature Biotechnology 20, 87-90, for Venus. Venus is a fluorescent protein obtained by substituting phenylalanine at position 46 of YFP with leucine. This protein achieves brightness that is 30 to 100 times greater than the conventional GFP in *Escherichia coli.* It achieves brightness that is 3 to 100 times greater than the conventional GFP in mammalian cells. Thus, Venus provides fluorescence that can sufficiently be detected with a common device.

Other fluorescent molecules that can be used in the present invention include: a yellow fluorescent protein derived from *Vibrio fischeri* Y- 1 strain; Peridinin-chlorophyll (a protein derived from dinoflagellate Symbiodinium sp.); a phycobili protein derived from marine cyanobacteria such as Synechoccus (e.g. phycoerythrin and phycocyanin); and oat phytochrome derived from oats, which is reconstructed with phycoerythrobilin. These fluorescent proteins are described in Baldwin T. O. et al., Biochemistry 29: 5509-5515 (1990), Morris B. J. et al., Plant Molecular Biology, 24: 673-677 (1994), and Wilbanks S. M. et al., J. Biol. Chem. 268: 1226-1235 (1993), Li et al., Biochemistry 34: 7923-7930 (1995), etc.

Examples of the combination of a donor protein with an acceptor protein used in the present invention may include CFP/YFP and BFP/GFP. However, examples are not limited thereto. A gene in which a fluorescent protein encodes a fusion protein can be constructed using a common genetic recombination technique that is known to persons skilled in the art.

The present invention is characterized in that a fluorescent protein which is a circularly permuted fluorescent protein obtained by substituting the amino acid sequence on the N-temrinal side of a wild type fluorescent protein or a mutant protein thereof, with that on the C-terminal side thereof, and which has a fluorescence peak wavelength substantially identical to that of a fluorescent protein that has not been subjected to the above circular permutation, is used as a donor protein and/or an acceptor protein.

That is to say, the circularly permuted fluorescent protein has the following amino acid sequences in the following order from the N-terminal side to the C-terminal side:
(1) an amino acid sequence from the n^{th} amino acid on the N-terminus of the original fluorescent protein to the C-terminus thereof (wherein n represents an interger of 2 or greater);
(2) a linker sequence consisting of 2 to 20 amino acids; and
(3) an amino acid sequence from the first amino acid on the N-terminus of the original fluorescent protein to the n-1^{th} amino acid thereof.

As described above, the operation to change the structure of a protein by substituting the amino acid sequence on the N-terminal side of the original protein with the amino acid sequence on the C-terminal side is also referred to as "circular permutation." In the present invention, circular permutation have been performed on the aforementioned various fluorescent proteins, so that novel fluorescent indicators having high dynamic ranges in FRET have been successfully produced.

The amino acid sequence of a linker sequence is not particularly limited, as long as a fusion fluorescent protein to be produced exhibits desired effects as a calcium ion indicator. The above amino acid sequence preferably mainly comprises an amino acid sequence having a relatively small side chain, and also, amino acids having a hydrophilic side chain are preferable. The number of amino acids is generally between approximately 2 and 20, preferably between approximately 3 and 10, and particularly preferably between approximately 5 and 10. A specific example of such a linker sequence is Gly-Gly-Ser-Gly-Gly, but is not limited thereto.

A position at which circular permutation is performed is not particularly limited, as long as the obtained circularly permuted fluorescent protein is able to have a fluorescence peak wavelength substantially identical to that of a fluorescent protein, which has not been subjected to the circular permutation. It is preferable that the amino acid sequence on the N-terminal side be substituted with the amino acid sequence on the C-terminal side, with respect to amino acid residues positioned at β-turn in the original amino acid sequence. Moreover, particularly preferably, the aforementioned amino acid residues positioned at the above β-turn are located at such a position that the dynamic range of the fluorescence of a circularly permuted fluorescent protein can be higher than that of a fluorescent protein, which has not been subjected to the circular permutation.

Specific examples of a fluorescent protein used in the present invention may include circularly permuted version of the fluorescent protein Venus, such as cp49Venus, cp157Venus, cp173Venus, cp195Venus, and cp229Venus, which were all produced in the examples of the present specification. However, examples are not limited thereto. cp49Venus, cp157Venus, cp173Venus, cp195Venus, and cp229Venus are produced by substituting the amino acid sequence on the N-terminal side with the amino acid sequence on the C-terminal side, with respect to Thr49 at amino acid No. 49 of the fluorescent protein Venus, Gln at amino acid No. 157 thereof, 173 at amino acid No. 173 thereof, Leu at amino acid No. 195 thereof, and Ile at amino acid No. 229 thereof, respectively.

Specific examples of the fluorescent indicator of the present invention may include YC3.20 (SEQ ID NO: 42), YC3.30 (SEQ ID NO: 43), YC3.60 (SEQ ID NO: 44), YC3.70 (SEQ ID NO: 45), and YC3.90 (SEQ ID NO: 46).

Specifically, the fluorescent indicator of the present invention adopts the following structure:
That is, there can be produced a fluorescent indicator, which further comprises a target peptide component and a linker component,
wherein the target sequence of the analytical substance further comprises a peptide-binding domain for allowing the target peptide component to bind thereto,
wherein the linker component allows the target sequence of the analytical substance to covalently bind to the target peptide component, and the target sequence and the target peptide component covalently bind to either the acceptor fluorescent molecular component or the donor fluorescent molecular component, and
wherein the analytical substance binding to the target sequence induces a change in the relative orientation of the target peptide component and the peptide-binding domain, and the relative positions or orientation of the donor molecular component and acceptor molecular component are then changed, thereby changing the efficiency of fluorescence resonance energy transfer (FRET).

In the present invention, a target sequence was produced by binding fluorescent molecules to both the N-terminus and C-terminus of a domain that causes a structural change due to Ca²⁺, and a fluorescent indicator was produced. Use of such a fluorescent indicator enables the monitoring of a change in the intracellular Ca²⁺ concentration.

The term "covalently binds" is used to mean a covalent bond, or other covalent connections between two molecules. An example of such a covalent connection may be a divalent component for connecting two molecules.

The term "target sequence" is used to mean an amino acid sequence capable of binding to an analytical substance. When a preferred target sequence binds to an analytical substance, its three-dimensional structure is changed.

The term "target peptide" is used to mean a peptide capable of binding to a target sequence. Such a target peptide is a partial sequence of a peptide binding to the target sequence.

The term "analytical substance" is used to mean a molecule or ion contained in a solution binding to a target sequence. Such an analytical substance changes the three-dimensional structure of a target sequence. An analytical substance may bind to a target sequence either reversibly or irreversibly.

The fluorescent molecular component preferably covalently binds to the amino terminus and carboxy terminus of the target sequence component. With such a configuration, the donor and acceptor fluorescent molecular components can move closely to each other, when an analytical substance binds thereto. Otherwise, the donor and acceptor components may also move such that they are separated from each other, when an analytical substance binds thereto. For example, such an acceptor component covalently binds to a target peptide component binding to a target sequence component, and the target peptide component covalently binds to the target sequence component via a linker component. Such a linker component is flexible, and the target peptide component can bind to the target sequence component via the linker component. The donor component is excited with light having appropriate intensity in the excitation spectrum thereof. The donor component emits the absorbed energy in the form of fluorescence. When the acceptor fluorescent molecular component exists at the position where it is able to quench the donor component in an exited state, the fluorescence energy is transferred to the acceptor component, thereby emitting fluorescence.

The FRET efficiency between the donor and acceptor fluorescent molecular components can be regulated by controlling the ability of the two fluorescent molecules to interact with each other. The properties of the target sequence component, target peptide component, and linker component also have effects on FRET and the response of the indicator to the analytical substance. Generally, it is desired that a significant change occur in the three-dimensional structure of the target sequence component.

The target sequence component is a protein the three-dimensional structure of which is changed due to the binding of the analytical substance, or a portion thereof. Examples of such a protein include calmodulin (CaM), cGMP-dependent protein kinase, a steroid hormone receptor (or a ligand-binding domain thereof), protein kinase C, an inositol-1,4,5-tirphosphate receptor, and recobelin (refer to Katzenellenbogen, J. A. & Katzenellenbogen, B. S. Chemistry & Biology 3: 529-536 (1996), and Ames, J. B. et al., Curr. Opin. Struct. Biol. 6: 432-438 (1996), for example). The target sequence component preferably binds to the target peptide as well as the analytical substance.

The target peptide component may include any given amino acid sequences shown in the following Table 1 and the portions thereof. However, the target peptide should be able to bind to the target sequence component. The target peptide may also be a partial sequence of a calmodulin-binding domain. The target peptide components shown in Table 1 are recognized by the target sequence component CaM (refer to Crivici, A. & Ikura, M. Annu. Rev. Biophys. Biomol. Struct. 24: 84-116 (1995), for example). The response of the fluorescent indicator to the analytical substance may be reinforced by modification of the target peptide component. Other target peptide components binding to other target sequences have already been known to persons skilled in the art.

**Table 1**

| Target | Sequence |
|---|---|
| SkMLCK (M13) | KRRWKKNFIAVSAANRFKKISSSGAL (SEQ ID NO: 1) |
| smMLCK (smMLCKp) | ARRKWQKTGHAVRAIGRLSS (SEQ ID NO: 2) |
| CaMKII | ARRKLKGAILTTMLATRNFS (SEQ ID NO: 3) |
| Caldesmon | GVRNIKSMWEKGNVFSS (SEQ ID NO: 4) |
| Calspermin | ARRKLKAAVKAVVASSRLGS (SEQ ID NO: 5) |
| PFK (M11) | FMNNWEVYKLLAHIRPPAPKSGSYTV (SEQ ID NO: 6) |
| Calcineurin | ARKEVIRNKIRAIGKMARVFSVLR (SEQ ID NO: 7) |
| PhK (PhK5) | LRRLIDAYAFRIYGHWVKKGQQQNRG (SEQ ID NO: 8) |
| (PhK13) | RGKFKVICLTVLASVRIYYQYRRVKPG (SEQ ID NO: 9) |
| Ca²⁺-ATPase (C28W) | LRRGQILWFRGLNRIQTQIKVVNAFSSS (SEQ ID NO: 10) |
| 59-kDa PDE | RRKHLQRPIFRLRCLVKQLEK (SEQ ID NO: 11) |
| 60-kDa PDE | TEKMWQRLKGILRCLVKQLEK (SEQ ID NO: 12) |
| NOS (NO-30) | KRRAIGFKKLAEAVKFSAKLMGQ (SEQ ID NO: 13) |
| Type I AC (AC-28) | IKPAKRMKFKTVCYLLVQLMHCRKMFKA (SEQ ID NO: 14) |
| *Bordetella pertussis* AC | IDLLWKIARAGARSAVGTEA (SEQ ID NO: 15) |
| Neuromodulin | KAHKAATKIQASFRGHITRKKLKGEKK (SEQ ID NO: 16) |
| Spectrin | KTASPWKSARLMVHTVATFNSIKE (SEQ ID NO: 17) |
| MARCKS | KKKKKRFSFKKSFKLSGFSFKKSKK (SEQ ID NO: 18) |
| F52 or MacMARKS | KKKKKFSFKKPFKLSGLSFKRNRK (SEQ ID NO: 19) |
| β -Adducin | KQQKEKTRWLNTPNTYLRVNVADEVQRNMGS (SEQ ID NO: 20) |
| HSP90a | KDQVANSAFQERLRKHGLEVI (SEQ ID NO: 21) |
| HIV-1 gp160 | YHRLRDLLLIVKRIVELLGRR (SEQ ID NO: 22) |
| BBMHBI | QQLATLIQKTYRGWRCRTHYQLM (SEQ ID NO: 23) |
| Dilute MHC | RAACIRIQKTIRGWLLRKRYLCMQ (SEQ ID NO: 24) |
| Mastoparan | INLKAALAKKIL (SEQ ID NO: 25) |
| Melittin | GIGAVLKVLTTGLPALISWIKRKRQQ (SEQ ID NO: 26) |
| Glucagon | HSQGTFTTSDYSKYLDSRRAQDFVQWLMNT (SEQ ID NO: 27) |
| Secretin | HSDGTFTSELSRLRDSARLQRLLQGLV (SEQ ID NO: 28) |
| VIP | HSDAVFTDNYTRLRKQMAVKKYLNSILN (SEQ ID NO: 29) |
| GIP | YADGTFISDYSAIMNKIRQQDFVNWLLAQQQKS (SEQ ID NO: 30) |
| Model peptide CBP2 | KLWKKLLKLLKKLLKLG (SEQ ID NO: 31) |

Explanation of abbreviated expressions
AC: adenylyl cyclase;
BBMHCI: brush-border myosin heavy chain-I;
CaMKII: calmodulin kinase II;
CBP2: calmodulin-binding peptide-2;
GIP: gastrin inhibitory peptide;
HIV- 1 gp160: human immunodeficiency virus envelope glycoprotein 160;
HSP: heat shock protein;
MARCKS: myristoylated alanine-rich C kinase substrate;
MHC: myosin heavy chain;
NOS: nitric oxide synthase;
PDE: phosphodiesterase;
PFK: phosphofructokinase;
PhK: phosphorylase kinase;
sk-, smMLCK: skeletal muscle and smooth muscle myosin light chain kinase; and
VIP: vasoactive intestinal peptide.

The length of a linker component is selected, such that it optimizes FRET and the speed and specificity regarding a change in the three-dimensional structure due to the binding of the analytical substance. Such a linker component preferably has a length and flexibility that are necessary for free interaction between the target sequence component and the target peptide component, which is capable of responding the concentration of the analytical substance. In order to optimize FRET effects, the mean distance between the donor and acceptor fluorescent molecular components is preferably between approximately 1 nm and approximately 10 nm, more preferably between approximately 1 nm and approximately 6 nm, and particularly preferably between approximately 1 nm and approximately 4 nm. If such a linker molecule is too short or too solid, the donor and acceptor molecular components cannot easily change the positions. In contrast, if such a linker molecule is too long, the target peptide component cannot efficiently bind to the target sequence component. Such a linker component is preferably a peptide component. A preferred linker component is a peptide consisting of 1 to 30 amino acid residues, and preferably 1 to 15 amino acid residues. The -Gly-Gly- linker is an example of such a linker.

A linker component may comprise a flexible spacer amino acid sequence. Such a linker component is described in Huston, J. S. et al., PNAS 85: 5879-5883 (1988), Whitlow, M. et al., Protein Engineering 6: 989-995 (1993), and Newton, D. L. et al., Biochemistry. 35: 545-553 (1996), for example.

Any target sequence may be used, as long as an analytical substance can bind to or act on the target sequence, so as to change the three-dimensional structure of an indicator. For example, a sequence that is recognized and cleaved with enzymes may also be used. For example, a substrate site of protease can be used as such a target sequence. When caspase 3 is used as protease, DEVD can be used as the amino acid sequence of a target sequence.

The fluorescent indicator may comprise a localizing sequence. Through such a localizing sequence, the indicator is fused with a preferred intracellular organelle target signal or with a localizing host protein, and it is thereby transferred to a specific site in a cell. A polynucleotide encoding the localizing sequence or signal sequence can be ligated to or fused with the 5'-terminus of a polynucleotide encoding the fluorescent indicator, so that a signal peptide can be positioned at the amino terminus of the obtained fusion polynucleotide or polypeptide.

In the case of a eukaryotic cell, it is considered that a signal peptide has a function of transporting a fusion polypeptide via the endoplasmic reticulum. The secretory protein is then transferred to the Goldi apparatus, and then to the secretory vesicle and the extracellular space, and preferably to the external environment. The signal peptide usable in the present invention may be a prepropeptide containing a proteolytic enzyme recognition site.

The localizing sequence may be a nucleus-localizing sequence, an endoplasmic reticulum-localizing sequence, a peroxisome-localizing sequence, a mitochondrion-localizing sequence, or a localizing protein. For example, the target sequence described in "Protein Targeting," Chapter 35, Stryer, L., Biochemistry (4th ed.). W H. Freeman, 1995, may also be used as a localizing sequence. The localizing sequence may also be a localizing protein. Specific examples of the localizing sequence include: a sequence targeting for the nucleus ((KKKRK) (SEQ ID NO: 32); a sequence targeting for the mitochondria (wherein the amino terminus is MLRTSSLFTRRVQPSLFRNILRLQST-) (SEQ ID NO: 33); a sequence targeting for the endoplasmic reticulum (KDEL (SEQ ID NO: 34) at the C-terminus) (wherein the signal sequence exists at the N-terminus); a sequence targeting for the peroxisome (SKF (SEQ ID NO: 35) at the C-terminus); a sequence targeting for prenylation or insertion into a cell membrane ([CaaX] CAAX (SEQ ID NO: 36), CC (SEQ ID NO: 37), CXC (SEQ ID NO: 38), or CCXX (SEQ ID NO: 39) at the C-terminus); a sequence targeting for the cytoplasmic side of a cell membrane (fusion with SNAP-25); and a sequence targeting for the Goldi apparatus (fusion with furin).

A fluorescent indicator can be produced in the form of a fusion protein by recombinant DNA techniques. The production of a fluorescent protein by recombination is carried out via the expression of nucleic acid encoding the protein. The nucleic acid encoding the fluorescent protein can be obtained by methods that have already been known to persons skilled in the art. For example, nucleic acid encoding a protein can be isolated from cDNA derived from *Aequorea aequorea* by PCR using primers based on the DNA sequence of an *Aequorea aequorea* green fluorescent protein. Various mutants of such a fluorescent protein can be produced by performing site-directed mutagenesis or random mutagenesis on nucleic acid encoding the fluorescent protein. Random mutagenesis can be carried out by performing PCR, using 0.1 mM MnCl or while destroying the balance of a nucleotide concentration.

The construction of an expression vector and the expression of a gene in cells transfected therewith can be carried out according to molecular cloning methods known to persons skilled in the art. The details of such molecular cloning methods are described in Sambrook et al., Molecular Cloning-A Laboratory Manual, Cold Spring Harbor Laboratory, Cold Spring Harbor, NY, (1989), and Current Protocols in Molecular Biology, F. M. Ausubel et al., eds., (Current Protocols, a joint venture between Greene Publishing Associates, Inc. and John Wiley & Sons, Inc., most recent Supplement).

Nucleic acid used for transfection of cells with a sequence encoding the expression of a polypeptide is generally an expression vector containing an expression regulatory sequence that is functionally ligated to a nucleotide sequence encoding the expression of a polypeptide. The expression "a nucleotide sequence encoding the expression of a polypeptide" is used herein to mean a sequence that generates a polypeptide as a result of the transcription and translation of mRNA. For example, a sequence containing intron is included in such a nucleotide sequence. The term "expression regulatory sequence" is used herein to mean a nucleic acid sequence that regulates the expression of nucleic acid, to which the expression regulatory sequence is functionally ligated. When such an expression regulatory sequence regulates and controls the transcription and translation of a nucleic acid sequence, it is functionally ligated to the nucleic acid sequence. Such an expression regulatory sequence may comprise a preferred promoter, enhancer, transcription terminator, initiation codon located before a protein-coding gene (that is, ATG), a splicing signal of intron, a termination codon, or the like.

An expression vector containing the coding sequence of a fluorescent indicator and an appropriate transcription and translation regulatory signal can be constructed by methods widely known to persons skilled in the art. Examples of such methods include *in vitro* recombination DNA technique, synthesis technique, *in vivo* recombination technique, and genetic engineering technique (refer to techniques described in Maniatis et al., Molecular Cloning A Laboratory Manual, Cold Spring Harbor Laboratory, N.Y, 1989, for example). Transformation of host cells with recombinant DNA can be carried out by common techniques that have widely been known to persons skilled in the art. When host cells are prokaryotic cells such as *Escherichia coli,* competent cells capable of taking in DNA can be produced from cells, which are recovered after the logarithmic growth phase and then treated by the CaCl₂ method widely known to persons skilled in the art. Otherwise, MgCl₂ or RbCl can also be used. Transformation can be carried out after the production of protoplasts of host cells, or by electroporation.

When host cells are eukaryotic cells, the calcium phosphate coprecipitation method, microinjection, electroporation, or the DNA transfection method such as insertion of a plasmid encapsulated into a liposome or a viral vector, can be applied. Eukaryotic cells can be transfected with a DNA sequence encoding the fusion polypeptide of the present invention and a foreign DNA molecule encoding an appropriate phenotype such as a herpes simplex thymidine kinase gene. Also, eukaryotic cells are transiently infected or transformed with a eukaryotic viral vector such as simian virus 40 (SV40) or bovine papilloma virus, so as to allow a protein to express therein (refer to Eukaryotic Viral Vectors, Cold Spring Harbor Laboratory, Gluzman ed., 1982). Preferably, eukaryotic cells are used as host cells.

As a method for isolating and purifying the polypeptide of the present invention that has been allowed to express in microorganisms or eukaryotic cells, any given common method can be used. For example, preparative chromatographic separation or immunological separation (including the use of a monoclonal or polyclonal antibody or an antigen) is applied.

In order to allow a sequence encoding the fluorescent indicator to express, various types of host/expression vector systems can be used. Examples of such a host/expression vector system include: bacteria transformed with a recombinant bacteriophage DNA, plasmid DNA or cosmid DNA expression vector which contains the sequence encoding the fluorescent indicator; yeast transformed with a recombinant yeast expression vector containing the sequence encoding the fluorescent indicator; plant cells infected with a recombinant viral expression vector containing the sequence encoding the fluorescent indicator (e.g. cauliflower mosaic virus (CaMV), tobacco mosaic virus (TMV)), or plant cells transformed with a recombinant plasmid expression vector (e.g. Ti plasmid) containing the sequence encoding the fluorescent indicator; an insect cell system infected with a recombinant viral expression vector (e.g. Baculovirus) containing the sequence encoding the fluorescent indicator; and an animal cell system infected with a recombinant viral expression vector (e.g. retrovirus, adenovirus, vaccinia virus) containing the sequence encoding the fluorescent indicator, but examples are not limited thereto.

Depending on the host/vector system used, appropriate transcription and translation elements (e.g. a constitutive or inducible promoter, a transcription-enhancer element, a transcription terminator, etc.) can be used in an expression vector (refer to Bitter et al., Methods in Enzymology 153: 516-544, 1987, for example). When an expression vector is cloned into bacteria for example, an inducible promoter, such as bacteriophage λ, plac, ptrp, or pL of ptac (a ptrp-lac hybrid promoter), can be used. When it is cloned into a mammalian cell system, a promoter derived from the genome of a mammalian cell (e.g. a metallothionein promoter) or a promoter derived from a mammalian virus (e.g. a retrovirus long terminal repeat, an adenovirus late promoter, a vaccinia virus 7.5 K promoter, etc.) can be used. It is also possible to transcribe an insertion sequence encoding the fluorescent indicator, using recombinant DNA or a promoter produced by synthesis techniques.

In the case of using bacteria, a large number of expression vectors can advantageously be selected depending on the intended use of the fluorescent indicator expressed therein. For example, when a large amount of fluorescent indicator is produced, it is preferable to use a vector that orders a large amount of expression of a fusion protein product that is easily purified. Such a vector is preferably processed such that it contains a cleavage site for supporting the recovery of the fluorescent indicator.

In the case of using yeast, a large number of vectors containing a constitutive or inducible promoter can be used. Please refer to Current Protocols in Molecular Biology, Vol. 2, Ed. Ausubel et al., Greene Publish. Assoc. & Wiley Interscience, Ch. 13, 1988; Grant et al., Expression and Secretion Vectors for Yeast, in Methods in Enzymology, Eds. Wu & Grossman, 31987, Acad. Press, N.Y., Vol. 153, pp.516-544, 1987; Glover, DNA Cloning, Vol. II, IRL Press, Wash., D.C., Ch. 3, 1986; Bitter, Heterologous Gene Expression in Yeast, Methods in Enzymology, Eds. Berger & Kimmel, Acad. Press, N.Y, Vol. 152, pp. 673-684, 1987; and The Molecular Biology of the Yeast Saccharomyces, Eds. Strathern et al., Cold Spring Harbor Press, Vols. I and II, 1982, for example. A constitutive yeast promoter such as ADH or LEU2 or an inducible promoter such as GAL can be used (Cloning in Yeast, Ch. 3, R. Rothstein In: DNA Cloning Vol. 11, A Practical Approach, Ed. DM Glover, IRL Press, Wash., D.C., 1986). Also, a vector that promotes incorporation of foreign DNA into the chromosome of yeast can be used.

In the case of using a plant expression vector, the expression of a sequence encoding the fluorescent indicator can be promoted with a promoter. For example, viral promoters such as CaMV 35S RNA and 19S RNA promoters (Brisson et al., Nature 310: 511-514, 1984) or the coat protein promoters of TMV (Takamatsu et al., EMBO J. 6:307-311, 1987) can be used. In addition, a plant promoter such as RUBISCO small subunit (Coruzzi et al., 1984, EMBO J. 3: 1671-1680; Broglie et al., Science 224: 838-843, 1984) or a heat shock promoter (e.g. soybean hsp 17.5-E or hsp 17.3-B (Gurley et al., Mol. Cell. Biol. 6: 559-565, 1986)) can also be used. These constructs can be introduced into plants using Ti plasmid, Ri plasmid, plant viral vector, direct DNA transformation, microinjection, electroporation, or the like. These techniques are described in Weissbach & Weissbach, Methods for Plant Molecular Biology, Academic Press, NY, Section VIII, pp. 421-463, 1988; and Grierson & Corey, Plant Molecular Biology, 2^{nd} Ed., Blackie, London, Ch. 7-9, 1988, for example.

It is also possible to allow the fluorescent indicator to express using an insect system. For example, a foreign gene can be allowed to express using *Autographa californica* nuclear polyhedrosis virus (AcNPV) as a vector. This virus grows in *Spodoptera frugiperda* cells. A sequence encoding the fluorescent indicator is cloned into the nonessential region of the above virus (e.g. a polyhedrosis gene), and it is placed under the control of the AcNPV promoter. When a sequence encoding the fluorescent indicator is correctly inserted, a polyhedrosis gene becomes inactivated, and a non-occlusive recombinant virus is generated. Thereafter, *Spodoptera frugiperda* cells are infected with such a recombinant virus, and the inserted gene can be allowed to express in the cells (refer to Smith et al., J. Viol. 46:584, 1983; and US Patent No. 4,215,051, for example).

Using a eukaryotic cell system, and preferably a mammalian cell expression system, it becomes possible to carry out a suitable post-translation modification on the expressed mammalian protein. Eukaryotic cells having a cell mechanism for the suitable processing, glycosylation, and phosphorylation of a primary transcript, and for the secretion of a gene product, can preferably be used as host cells for expression of the fluorescent indicator. Examples of such a host cell line include CHO, VERO, BHK, HeLa, COS, MDCK, Jurkat, HEK-293; and WI38, but examples are not limited thereto.

A mammalian cell line that orders expression using a recombinant virus or viral element can be constructed. When an adenovirus expression vector is used for example, a sequence encoding the fluorescent indicator can be ligated to an adenovirus transcription/translation regulatory complex (e.g. a late promoter and 3 leader sequences, etc.) This chimeric gene can be inserted into adenovirus genome by *in vitro* or *in vivo* recombination. By inserting the gene into the nonessential region (e.g. E1 or E3 region) of the virus genome, a recombinant virus capable of surviving in the infected host and allowing the fluorescent indicator to express therein can be obtained (refer to Logan & Shenk, Proc. Natl. Acad. Sci. USA, 81: 3655-3659, 1984, for example). Otherwise, a vaccinia virus 7.5K promoter can be used (refer to Mackett et al., Proc. Natl. Acad. Sci. USA , 79: 7415-7419, 1982; Mackett et al., J. Virol. 49: 857-864, 1984; Panicali et al., Proc. Natl. Acad. Sci. USA 79: 4927-4931, 1982, for example). It is also possible to use a vector based on a bovine papilloma virus having replication ability as an extrachromosomal element (Sarver et al., Mol. Cell. Biol. 1: 486, 1981). Immediately after this DNA has been introduced into mouse cells, a plasmid replicates approximately 100 to 200 copies per cell. In order to transcribe the inserted cDNA, it is not necessary for the plasmid to be incorporated into the chromosome of the host. Thus, a high level of expression can be realized. By incorporating a selective marker such as a neo gene into a plasmid, these vectors can be used for stable expression. Alternatively, retrovirus genome is modified, and the modified genome can be used as a vector capable of inducing or ordering the expression of the fluorescent indicator gene in host cells (Cone & Mulligan, Proc. Natl. Acad. Sci. USA, 81: 6349-6353, 1984). A high level of expression can be achieved by using an inducible promoter such as a metalothionine IIA promoter or a heat shock promoter.

In order to produce a recombinant protein at a high yield for a long period of time, stable expression is preferable. Host cells can be transformed with the cDNA of a fluorescent indicator, which is regulated with suitable expression regulatory elements (e.g. a promoter, an enhancer, a sequence, a transcription terminator, a polyadenylation site, etc.) and a selective marker, instead of using an expression vector containing a replication origin of virus. A selective marker contained in a recombinant plasmid imparts a resistance to selection, so that a cell stably incorporates the plasmid into the chromosome thereof, and the cell then grows and forms a colony. The colony is then subjected to cloning, so as to establish a cell line. For example, after introduction of foreign DNA, the recombinant cells are allowed to proliferate in a rich medium for 1 or 2 days, and then the medium is exchanged with a selective medium. A large number of selective systems can be used. For example, a herpes simplex thymidine kinase gene (Wigler et al., Cell, 11: 223, 1977), a hypoxanthine guanine phosphoribosyltransferase gene (Szybalska & Szybalski, Proc. Natl. Acad. Sci. USA, 48:2026, 1962), and an adenine phosphoribosyltransferase gene (Lowy et al., Cell, 22: 817, 1980) are used in tk-, hgprt-, and aprt-cells, respectively. Moreover, antimetabolite resistance can be used as a base for the selection of a dhfr gene imparting a resistance to methotrexate (Wigler et al., Proc. Natl. Acad. Sci. USA, 77: 3567, 1980; O'Hare et al., Proc. Natl. Acad. Sci. USA, 8: 1527, 1981), a gpt gene imparting a resistance to mycophenolic acid (Mulligan & Berg, Proc. Natl. Acad. Sci. USA, 78: 2072, 1981), a neo gene imparting a resistance to aminoglucoside G-418 (Colberre-Garapin et al., J. Mol. Biol., 150:1, 1981), and a hygro gene imparting a resistance to hydromycin (Santerre et al., Gene, 30: 147, 1984).

In recent years, other selective genes have further been reported. Examples of such selective genes include: trpB that enables cells to use indole instead of tryptophan; hisD that enables cells to use histinol instead of histidine (Hartman & Mulligan, Proc. Natl. Acad. Sci. USA, 85:8047, 1988); and ODC (ornithine decarboxylase) that imparts a resistance to 2-(difluoromethyl)-DL-ornithine that is an ornithine decarboxylase inhibitor (McConlogue L., In: Current Communications in Molecular Biology, Cold Spring Harbor Laboratory, ed., 1987).

A DNA sequence encoding the polypeptide of the fluorescent indicator of the present invention can be allowed to express *in vitro* by introducing the DNA into suitable host cells. That is to say, the recombinant fluorescent protein of the present invention can be produced by allowing nucleic acid to express in prokaryotic cells such as *Escherichia coli* or in eukaryotic cells such as yeast or mammalian cells.

A construct may comprise a tag used for easy isolation of the fluorescent indicator. For example, a polyhistidine tag consisting of 6 histidine residues may be added to the amino terminus of the fluorescent protein. With such a polyhistidine tag, it becomes possible to easily isolate the protein in a single operation by nickel chelate chromatography.

The fluorescent indicator of the present invention is preferably a fusion protein produced by the recombinant DNA technique. Herein, a single polypeptide comprises a donor component, a peptide linker component, and an acceptor component. The donor component may be positioned on the amino terminal side with respect to the acceptor component in the polypeptide. Such a fusion protein generally has the following structure: (amino terminus) a donor fluorescent protein - a peptide linker component - an acceptor fluorescent protein (carboxy terminus). Alternatively, the donor component may also be positioned on the carboxy terminal side with respect to the acceptor component in the fusion protein. Such a fusion protein generally has the following structure: (amino terminus) an acceptor fluorescent protein - a peptide linker component ― a donor fluorescent protein (carboxy terminus). Moreover, a fusion protein containing an amino acid sequence (e.g. a polyhistidine tag, etc.) added to the amino terminus and/or carboxy terminus may also be included in the present invention.

The fluorescent indicator encoded by recombinant nucleic acid comprises a sequence encoding the expression of a donor fluorescent protein, an acceptor fluorescent protein, and a peptide linker component. Each constitutive element is selected, such that when a donor component is excited as a result of the expression of a fusion protein, donor and acceptor components exhibit FRET. Recombinant nucleic acid can be incorporated into an expression vector containing an expression regulatory sequence that is functionally ligated to the recombinant nucleic acid. An expression vector comprises a suitable promoter, replication sequence, marker, or the like, so that it can function in prokaryotic cells or eukaryotic cells.

Host cells can be transfected with such an expression vector, so as to allow recombinant nucleic acid to express therein. Host cells can be selected for a high level of expression of nucleic acid, from which a fluorescent indicator fusion protein is then purified. *Escherichia coli (E. coli)* is useful for such purpose. In addition, host cells may also be either other prokaryotic cells or eukaryotic cells. Such cells may be either cultured cells or *in vivo* cells. The present invention will be specifically described in the following examples. However, the examples are not intended to limit the scope of the present invention.

### EXAMPLES

### A. Method

### (1) Gene construction

The cDNAs of the 5' portions of the cpVenus variants were amplified by PCR using sense primers containing a *Bam*HI site and reverse primers containing the sequence encoding the linker (GGSGG) between the natural N- and C-termini. The cDNAs of their 3' portions were extended by PCR at the 5' end with the sequence encoding the linker and at the 3' end with the sequence containing an *Eco*RI site. The entire cDNAs of the cp Venus variants were amplified using a mixture of the two PCR products with the *Bam*HI and *Eco*RI containing primers. The restricted products were cloned in-frame into the *Bain*HI/*Eco*RI sites of pRSET_{B} (Invitrogen), yielding cp49Venus, cp157Venus, cp173Venus, cp195Venus, and cp229Venus. Then the 5' end of the cDNA of cp49Venus, cp157Venus, cp173Venus, cp195Venus, or cp229Venus was modified by PCR to have a *Sac*I site. This N-terminal EL (Glu-Leu) sequence encoded by the *Sac*I recognition site was followed in the five variants by a Met residue and then Thr49, Gln157, Asp173, Leu195, and Ile229, respectively. The *Sac*I*lEco*RI fragments were substituted for the gene encoding Venus in YC3.12/pRSET_{B} to generate YC3.20, YC3.30, YC3.60, YV3.70, and YC3.90, respectively. YC2.60 and YC4.60 were generated from YC3.60 by exchanging the CaM domains. For mammalian expression, the cDNAs of YC3.12 and YC3.60 were subcloned into pcDNA3 (Invitrogen). To localize YC3.60 beneath the plasma membrane, the CAAX box of Ki-Ras was fused to the carboxyl terminus of YC3.60 through a linker sequence (GTGGSGGGTGGSGGGT)(SEQ ID NO:40).

### (2) Protein expression, in vitro spectroscopy, Ca²⁺ and pH titrations

Recombinant YC proteins with N-terminal polyhistidine tags were expressed in *Escherichia coli* [JM109(DE3)] at room temperature, purified, and spectroscopically characterized as previously described (Miyawaki A. et al., (1997) Nature 388, 882-887). Steady-state fluorescence polarization was measured using BECON (Takara) using a 440DF20 excitation filter and a 535DF25 emission filter. Ca²⁺ titrations were performed by reciprocal dilution of Ca²⁺-free and Ca²⁺-saturated buffers prepared by using 0,09bis(2-aminoethyl)ethyleneglycol-N,N,N9,N9tetraacetic acid (EGTA), N-(2-hydroxyethyl)ethylenediamine-N,N9,N9-triacetic acid (EDTA-OH), or nitrilotriacetic acid (NTA). pH titrations were performed by using a series of buffers prepared with pHs ranging from 5.8 to 8.4 as previously described (Nagai T, et al., (2001) Proc. Natl. Acad. Sci. USA 98, 3197-3202).

### (3) Cell culture and transfection

HeLa cells were grown in Dulbecco's modified Eagle's medium containing 10% heat-inactivated fetal calf serum. Cells were transfected with expression vectors encoding YC3.60 or YC3.12 using Superfect (QIAGEN).

### (4) Imaging

Between two and four days after transfection, HeLa cells in Hanks' balanced salt solution buffer (GIBCO) were subjected to imaging. Wide field fluorescence observations were performed on an IX-70 inverted microscope using a UApo 40x, 1.35 NA oil-immersion objective (Olympus). Dual-emission imaging with YCs used a 440DF20 excitation filter, a 455DRLP dichroic mirror and two emission filters (480DF30 for CFP and 535DF25 for YFP) alternated using a filter changer (Lambda 10-2, Sutter instruments). Interference filters were obtained from Omega Opical. Fluorescence emission from YCs was imaged using a cooled CCD camera (Cool SNAP fx, Roper Scientific). Image acquisition and analysis were performed using Metamorph/Metafluor5.0 software (Universal Imaging). Video rate confocal FRET images were acquired using an IX-71 equipped with a PlanApo 60x, 1.4 NA oil-immersion objective (Olympus), a spinning disc-type confocal unit (CSU21, Yokogawa), a diode-pumped solid state laser (430nm) (Hitachi), and a 3CCD color camera (ORCA-3CCD, Hamamatsu Photonics). Image acquisition and analysis were performed using Aquacosmos 2.5 software (Hamamatsu Photonics).

### B. Results

### (1) Structures and spectral properties of YC3.12 and the new YC variants (Figure 1)

Circular permutation was conducted on Venus using a peptapeptide linker GGSGG to connect the natural N and C termini. New termini were introduced into surface-exposed loop regions of the β-barrel. cp49Venus, cp157Venus, cp173Venus, cp195Venus, and cp229Venus were given new N termini at Thr49, Gln157, Asp173, Leu195, and Ile229, respectively. When expressed in bacteria and mammalian cultured cells, they matured efficiently and were resistant to acidification to a similar extent as their parent protein Venus. The use of these cpVenus proteins in addition to Venus would create significant variation in the relative spatial orientation of YFP within the YC complex, since Met1, Thr49, Gln153, Asp173, Leu195, and Ile229 reside at different sites on the β-barrel. Thr49 and Asp173 are particularly far removed at the opposite end of the β-barrel from the other residues (Figure 1A).

As a parent YC, YC3.12 (Nagai T. et al., (2002) Nat. Biotechnol. 20, 87-90) was used initially due to its monophasic Ca²⁺ sensitivity. It has a mutation of a conserved glutamate (E104) in the third Ca²⁺-binding site of CaM and belongs to the YC3 group. Venus in YC3.12 was replaced with cp49Venus, cp157Venus, cp173Venus, cp195Venus, and cp229Venus to generate YC3.20, YC3.30, YC3.60, YC3.70, and YC3.90, respectively (Figure 1B). All of these new YCs were expressed efficiently and folded in bacteria, similar to YC3.12. Next, their Ca²⁺ sensitivity was examined by *in vitro* experiments. Remarkably, YC3.60 gave a several fold increase in the emission ratio of YFP to CFP. between zero and saturating Ca²⁺ concentrations, while YC3.30, YC3.70, and YC3.90 showed a similar dynamic range to that of YC3.12. YC3.20 showed only a slight response to Ca²+ (Figure 1C). Although the substitution of cp173Venus for Venus generally favored the FRET from CFP, this effect was much more striking in the complex's Ca²⁺-saturated form *(R*_{*max*}*:* 1.8 (YC3.12) vs. 9.3 (YC3.60)) than in its Ca²⁺-depleted form *(R*_{*min*}*:* 0.87 (YC3.12) vs. 1.4 (YC3.60)) (Table 2A). To examine the relative angle between the chromophores of CFP and YFP, steady state polarization (anisotropy) was measured with excitation of CFP at 440 nm and emission of YFP at 535 nm. The Ca²⁺-dependent decrease in anisotropy correlated with the increase in the emission ratio of YFP to CFP (Figure 1D).

The emission ratio (535/480) of YC3.60 showed a monophasic Ca²⁺-dependency with an apparent dissociation constant (K'_{d}) of 0.25 µM and a Hill constant *(n)* of 1.7 (Figure 1E, circles). To change the Ca²⁺ affinity of YC3.60, the mutated CaM was replaced with either wild-type CaM or a CaM containing a mutation in the first Ca²⁺-binding loop (E31Q) (Miyawaki A. et al., (1997) Nature *388,* 882-887). The resulting YCs belong to the YC2 and YC4 groups, and are called YC2.60 and YC4.60, respectively. YC2.60 showed a nearly monophasic response (*K'*_{d}, 40 nM; *n,* 2.4). There was a tiny depression on the titration curve at 0.2 - 0.3 µM (Figure 1E, triangles), reminiscent of the biphasic Ca²⁺-sensitivity of the original CaM-M13 hybrid protein (Miyawaki A. et al., (1997) Nature *388,* 882-887; and Porumb T. et al., (1994) Protein Eng. 7, 109-115). As described previously (Miyawaki A. et al., (1997) Nature *388,* 882-887; and Porumb T. et al., (1994) Protein Eng. 7, 109-115), E31Q in YC4.60 gave a significantly lower Ca²⁺ affinity with a clear biphasic response (*K*'ₐ, 58 nM; *n*, 1.7; *K*'_{d}, 14.4 µM; *n*, 0.87) (Figure 1E, squares). The high dynamic range achieved in YC3.60 (570%) was preserved in YC2.60, but slightly attenuated in YC4.60 (dynamic range, 360%). The high- and low-affinity components of YC4.60 contributed to 41% and 59% of the response. Since the cpVenus proteins displayed similar acid sensitivity (pKa, 6.0) to EYFP-V68L/Q69K (EYFP.1) or Venus, YC3.60 was expected to be as pH-resistant as YC3.1 and YC3.12. The pH titration curves in Figure 1F show that the YFP/CFP ratio does not change significantly in the presence and absence of Ca²⁺ over a physiological range of pH, from 6.5 to 8.2. Compared with YC3.1 and YC3.12, however, YC3.60 gives a large Ca²⁺-dependent response that overwhelms the noise due to the pH change, resulting in a much better signal-to-noise ratio. The properties of YC variants are summarized in Table 2A and 2B. Table 2A shows Ca²⁺-responses of the conventional and new YC variants. Table 2B shows affinities for Ca²⁺ of YC3.60 and its derivatives.

**Table 2**

| Table2A | | | | | |
|---|---|---|---|---|---|
| | *R*ₘᵢₙ | *R*ₘₐₓ | dynamic range (%) | anisotropy | |
| | | | | -Ca²⁺ | +Ca²⁺ |
| YC3.12 | 0.9 | 1.8 | 100 | 0.23 | 0.17 |
| YC3.20 | 1.3 | 1.4 | 10 | 0.06 | 0.10 |
| YC3.30 | 1.1 | 2.6 | 140 | 0.16 | 0.07 |
| YC3.60 | 1.4 | 9.3 | 560 | 0.12 | - 0.05 |
| YC3.70 | 1.2 | 2.4 | 100 | 0.15 | 0.09 |
| YC3.90 | 1.0 | 1.7 | 70 | 0.17 | 0.10 |

| Table2B | | | | | |
|---|---|---|---|---|---|
| | K'd(nM) | fraction (%) | Hill coef | | |
| YC2.60 | 40 | ― | 2.4 | | |
| YC3.60 | 250 | ― | 1.7 | | |
| YC4.60 | 60 14000 | 40 60 | 1.7 0.9 | | |

### (2) Comparative measurements of Ca²⁺ dynamics in HeLa cells expressing YC3.60 and YC3.12 (Figure 2)

The superiority of YC3.60 to YC3.12 was demonstrated clearly in experiments in which we monitored cytosolic free Ca²⁺ concentrations ([Ca²⁺]_{c}s) in HeLa cells. HeLa cells transfected with the same amount of cDNAs encoding either YC3.60 or YC3.12 produced equally bright fluorescence signals in the cytosolic compartment (Figures 2A and 2B, rescpectively). Figures 2C and 2D show the time courses of the spatially averaged YFP/CFP ratios from HeLa cells expressing YC3.60 and YC3.12, respectively. YC3.60 clearly gives a much larger responses to a supramaximal dose of ATP (30 µM) and nearly 6-fold larger ratios of *R*ₘₐₓ to *R*ₘᵢₙ than does YC3.12. This comparison also indicates a difference in Ca²⁺ affinity between the two YCs (*K*'_{d} = 0.25 µM for YC3.60 vs. 1.5 µM for YC3.12). It should be noted that both the *R*ₘₐₓ and Rₘᵢₙ values of YC3.60 did not vary among the three cells shown in Figure 3A and HeLa cells in four other experiments conducted with the same microscopic system (*R*ₘₐₓ*,* 8.06 ± 0.16, n = 12; *R*ₘᵢₙ, 1-37 ± 0.10, n = 12), in contrast to the cell-to-cell variation seen in the corresponding values for YC3.12.

### (3) Confocal imaging of [Ca²⁺]_{c} and [Ca²⁺]ₚₘ in HeLa cells using YC3.60

The large dynamic range and brightness of YC3.60 enables substantial improvement in both temporal and spatial resolution of [Ca²⁺]_{c} imaging. For fast and simultaneous acquisition of the YFP and CFP images, a color camera composed of three CCD chips (RGB: red, green, and blue) and a prism was used. For imaging, the YFP and CFP images were captured by the G and B chips, respectively. Also, to improve spatial resolution along the z-axis, a spinning disk unit was placed in front of the camera. A confocal real-color image of YC3.60-expressing HeLa cells is shown in Figure 3B. The fluorescence was uniformly distributed in the cytosolic compartment but excluded from the mitochondria as well as the nucleus. A series of ratio images in pseudo-color acquired at video rate (Figure 3A) shows how the increase in [Ca²⁺]_{c} appeared and propagated within the individual cells after stimulation with histamine. The propagation velocity was calculated to be 30 µm/s from the time courses of [Ca²⁺]_{c} at 6 aligned ROIs (regions of interest) in one cell (Figures 3B and 3C).

To demonstrate the benefits of YC3.60, YC3.60 was targeted to the plasma membrane by fusing the membrane anchor sequence of Ki-Ras to the C-terminus of the indicator (YC3.60ₚₘ). Using similar membrane-targeting approaches, conventional YCs have not been able to monitor the Ca²⁺ dynamics beneath the plasma membrane. The fluorescence of YC3.60ₚₘ was distributed to the periphery and filopodial structures (Figure 3D). The free Ca²⁺ concentration beneath the plasma membrane ([Ca²⁺]ₚₘ) was measured quantitatively (Figure 3E). [Ca²⁺]ₚₘ before the application of histamine was slightly higher than the basal level of [Ca²⁺]_{c}, which may support the notion that there exist microdomains of high [Ca²⁺] in the submicroscopic environment (Marsault R. et al., (1997) EMBO J. *16*, 1575-1581). Similar changes in [Ca²⁺]ₚₘ were also observed in the filopodia structures (Figure 3F).

### INDUSTRIAL APPLICABILITY

The fluorescent indicator of the present invention makes it possible to diversify the relative positional relationship between an energy donor and an energy acceptor, by using a fluorescent protein that has been subjected to circular permutation. As a result, it becomes possible to increase the dynamic ranges of various fluorescent indicators. Moreover, the fluorescent indicator of the present invention can be produced *in situ* by introduction of a gene into a cell or a living body. Thus, it is not necessary that a large amount of soluble recombinant protein be allowed to express and be purified, that the thus obtained protein be purified and labeled *in vitro,* and that it be then returned to a cell by microinjection. Furthermore, the fluorescent indicator of the present invention is able to be targeted to a cell structure.

## Claims

1. A fluorescent indicator, which has a structure such that a donor fluorescent protein and an acceptor fluorescent protein bind to both termini of the target sequence of an analytical substance, wherein the analytical substance binds to or acts on said target sequence, so that the three-dimensional structure of the indicator can be changed, thereby generating fluorescence resonance energy transfer (FRET), said fluorescent indicator being **characterized in that** the said donor fluorescent protein and/or said acceptor fluorescent protein are circularly permuted fluorescent proteins obtained by substituting the amino acid sequence on the N-terminal side of a wild-type fluorescent protein or a mutant protein thereof with the amino acid sequence on the C-terminal side thereof, and **in that** the thus obtained fluorescent proteins have a fluorescent peak wavelength substantially identical to that of a florescent protein, which has not yet been subjected to the circular permutation.

2. The fluorescent indicator according to claim 1 wherein the fluorescent protein is GFP, CFP, YFP, RFP, BFP, or a mutant thereof.

3. The fluorescent indicator according to claim 1 or 2 wherein the donor fluorescent protein is CFP or a mutant thereof, and the acceptor fluorescent protein is YFP or a mutant thereof.

4. The fluorescent indicator according to any of claims 1 to 3 wherein the donor fluorescent protein and/or the acceptor fluorescent protein are circularly permuted fluorescent proteins, which are obtained by substituting the amino acid sequence on the N-terminal side with the amino acid sequence on the C-terminal side with respect to amino acid residues positioned in a β turn in the amino acid sequence of a wild-type fluorescent protein or a mutant protein thereof.

5. The fluorescent indicator according to claim 4 wherein the amino acid residues positioned in the β turn are amino acid residues which are positioned such that the dynamic range of the fluorescence of a fluorescent protein can be increased.

6. The fluorescent indicator according to any of claims 1 to 5 wherein the acceptor fluorescent protein is a circularly permuted version of the fluorescent protein Venus.

7. The fluorescent indicator according to claim 6 wherein the circularly permuted versions of Venus are cp49Venus, cp157Venus, cp173Venus, cp195Venus, or cp229Venus.

8. The fluorescent indicator according to any of claims 1 to 7 which further comprises a target peptide component and a linker component,
wherein the target sequence of the analytical substance further comprises a peptide-binding domain for allowing the target peptide component to bind thereto,
wherein the linker component allows the target sequence of the analytical substance to covalently bind to the target peptide component, and the target sequence and the target peptide component covalently bind to either the acceptor fluorescent molecular component or the donor fluorescent molecular component, and
wherein the analytical substance binding to the target sequence induces a change in the relative positions or orientations of the target peptide component and the peptide-binding domain, and the relative positions or orientations of the donor molecular component and acceptor molecular component are then changed, thereby changing the efficiency of fluorescence resonance energy transfer (FRET).

9. The fluorescent indicator according to any of claims 1 to 8 wherein the target sequence is calmodulin, cGMP-dependent protein kinase, a steroid hormone receptor, a ligand-binding domain of a steroid hormone receptor, protein kinase C, inositol-1,4,5-triphosphate receptor, or recobelin.

10. The fluorescent indicator according to claim 9 wherein the target sequence is calmodulin.

11. The fluorescent indicator according to claim 8 wherein the target peptide component is skeletal muscle myosin light chain kinase (skMLCKp), smooth muscle light chain kinase (smMLCK), calmodulin kinase II (CaMKII), caldesmon, calspermine, phosphofructokinase, calcineurin, phosphorylase kinase, Ca²⁺-ATPase, 59 Kda phosphodiesterase (PDE), 60 Kda phosphodiesterase (PDE), nitric oxide synthase, type I adenylyl cyclase, *Bordetella pertussis* adenylyl cyclase, neuromodulin, spectrin, myristoylated alanine-rich C kinase substrate (MARCKS), MacMARCKS(F52), b-Adducin, heat shock protein HSP90a, human immunodeficiency virus envelope glycoprotein 160 (HIV-1 gp160), brush-boarder myosin heavy chain-I (BBMHBI), dilute myosin heavy chain (MHC), mastoparan, melittin, glucagon, secretin, vasoactive intestinal peptide (VIP), gastrin inhibitory peptide (GIP), or a calmodulin-binding domain of calmodulin-binding peptide-2 (Model peptide CBP2).

12. The fluorescent indicator according to claim 8 wherein the linker component is a peptide component consisting of 1 to 30 amino acid residues.

13. The fluorescent indicator according to any of claims 1 to 12 which further comprises a localizing sequence.

14. The fluorescent indicator according to any of claims 1 to 13 wherein the localizing sequence is a nucleus-localizing sequence, an endoplasmic reticulum-localizing sequence, a peroxisome-localizing sequence, a mitochondrion-localizing sequence, a Goldi apparatus-localizing sequence, or a cell membrane-localizing sequence.

15. A fluorescent indicator having the amino acid sequence shown in any one of SEQ ID NOS: 42, 43, 44, 45, and 46.

16. A method for detecting or measuring an analytical substance contained in a sample, which comprises:
(1) a step of allowing a sample to come into contact with the fluorescent indicator of any of claims 1 to 15;
(2) a step of exciting a donor component; and
(3) a step of measuring the level of fluorescence resonance energy transfer in the sample that corresponds to the concentration or activity of the analytical substance contained in the sample.

17. The method according to claim 16 wherein the sample is a living cell, and said contact step includes incorporation of the fluorescent indicator into such a living cell.

18. The method according to claim 17 wherein the step of incorporating the fluorescent indicator into a cell comprises transfection of the cell with an expression vector which contains an expression regulatory sequence that is functionally ligated to a nucleic acid sequence encoding the expression of the fluorescent indicator.

19. A nucleic acid encoding the fluorescent indicator of any of claims 1 to 15.

20. An expression vector containing the nucleic acid of claim 19.

21. A transformant having the nucleic acid of claim 19 or the expression vector of claim 20.
